# EUROPEAN PATENT APPLICATION

(11) **EP 4 273 137 A1**
(43) Date of publication of application: **08.11.2023**
(21) Application number: 21914669.3
(22) Date of filing: 30.12.2021
(51) Int. Cl.: C07D 401/06, C07D 401/02, C07D 403/02, C07D 403/06, A61K 31/454, A61P 13/12

(54) **SERIES OF PIPERIDINE-SUBSTITUTED BENZOIC ACID COMPOUNDS, AND USE THEREOF**

(30) Priority: 30.12.2020 CN 202011610857; 11.03.2021 CN 202110266011; 02.07.2021 CN 202110751316; 22.10.2021 CN 202111234085
(71) Applicant: Medshine Discovery Inc., Nanjing, Jiangsu 210032 (CN)
(72) Inventor: CHEN, Kevin X, Shanghai 200131 (CN); ZHANG, Li, Shanghai 200131 (CN); GUI, Houze, Shanghai 200131 (CN); ZHANG, Haoyu, Shanghai 200131 (CN); ZHANG, Huiyu, Shanghai 200131 (CN); HU, Guoping, Shanghai 200131 (CN); LI, Jian, Shanghai 200131 (CN); CHEN, Shuhui, Shanghai 200131 (CN)
(74) Representative: RGTH
(86) International application number: PCT/CN2021/143257
(87) International publication number: WO 2022/143940

(57) **Abstract**

A series of piperidine-substituted benzoic acid compounds and the use thereof, and specifically disclosed is a compound represented by formula (I) and a pharmaceutically acceptable salt thereof.

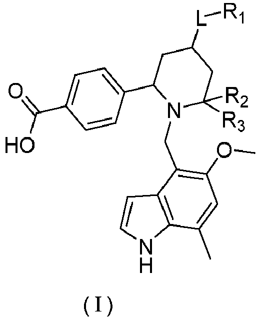

## Description

The present invention claims the right of the following priorities:
CN202011610857.7, filed on December 30, 2020;
CN202110266011.4, filed on March 11, 2021;
CN202110751316.4, filed on July 2, 2021;
CN202111234085.6, filed on October 22, 2021.

### TECHNICAL FIELD

The present disclosure relates to a series of piperidine-substituted benzoic acid compounds, specifically to a compound represented by formula (I) and a pharmaceutically acceptable salt thereof.

### BACKGROUND

Immune diseases are caused by an imbalance in immune regulation which affects the body's immune response. The complement system is an important component of the immune system and contains a group of proteins that are normally present in an inactive state, known as complements. Complements can be activated by substances such as lipopolysaccharide, polysaccharide, peptidoglycan, teichoic acid, and aggregated IgA and IgG4 in the alternative activation pathway, mediating immune responses and inflammatory reactions. Herein, an activator directly activates C3 and then completes a chain reaction of the components of C5 to C9. Complement factor B, also known as C3 activator precursor, can be cleaved into two fragments Ba and Bb by complement factor D, and Bb combines with C3b to form a C3 convertase of the alternative pathway. Complement factor B acts on the AP pathway. Inhibiting the activity of factor B can prevent the activation of the API pathway without interfering with the CP and LP pathways, and can avoid increasing the risk of infection due to the inhibition of the complement system.

There is no small-molecule factor B inhibitor on the market yet. Factor B inhibitor LNP023 from Novartis is in the phase III clinical research stage for the treatment of PNH, IgAN, C3G, and other diseases. Therefore, there is an urgent need in the art to develop a novel small-molecule factor B inhibitor of the complement system for the treatment of various diseases caused by complement abnormalities through increased clinical research and validation.

### CONTENT OF THE PRESENT INVENTION

The present disclosure provides a compound represented by formula (I) or a pharmaceutically acceptable salt thereof, 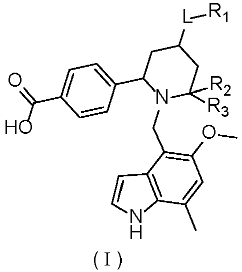 wherein
L is selected from a single bond, NR₄, and O;
R₁ is selected from fluorine, chlorine, C₁₋₅ alkyl, C₃₋₆ cycloalkyl, 3- to 6-membered heterocycloalkyl, -C₁₋₃ alkyl-C₁₋₃ alkoxy, -C₁₋₃ alkyl-C₃₋₆ cycloalkyl, and -C₁₋₃ alkyl-3- to 6-membered heterocycloalkyl, and the C₁₋₅ alkyl, C₃₋₆ cycloalkyl, 3- to 6-membered heterocycloalkyl, -C₁₋₃ alkyl-C₁₋₃ alkoxy, -C₁₋₃ alkyl-C₃₋₆ cycloalkyl, and -C₁₋₃ alkyl-3- to 6-membered heterocycloalkyl are each independently and optionally substituted by 1, 2, or 3 Rₐ groups;
R₂, R₃, and R₄ are each independently selected from H and C₁₋₅ alkyl, and the C₁₋₅ alkyl is optionally substituted by 1, 2, or 3 R_{b} groups;
each Rₐ and R_{b} are independently selected from H, F, Cl, Br, and I;
provided that R₂ and R₃ are not simultaneously selected from H when R₁ is selected from unsubstituted C₁₋₅ alkyl.

In some embodiments of the present disclosure, the compound or the pharmaceutically acceptable salt thereof is selected from: 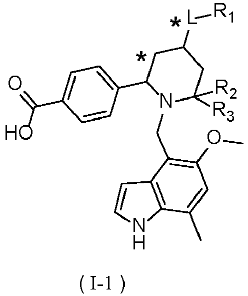 wherein
L, R₁, R₂, and R₃ are as defined in the present disclosure;
the carbon atom with "*" is a chiral carbon atom, which exists in a (R) or (S) single enantiomer form or a (R) or (S) single enantiomer-rich form.

In some embodiments of the present disclosure, the compound or the pharmaceutically acceptable salt thereof is selected from: 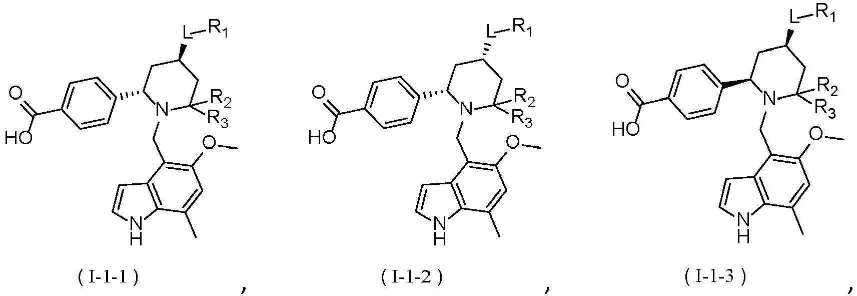 and 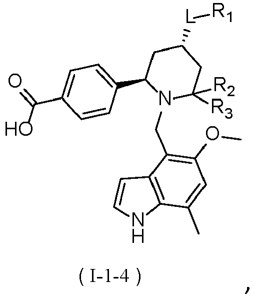 wherein L, R₁, R₂, and R₃ are as defined in the present disclosure.

In some embodiments of the present disclosure, the R₁ is selected from C₃₋₆ cycloalkyl, 3- to 6-membered heterocycloalkyl, -C₁₋₃ alkyl-C₁₋₃ alkoxy, -C₁₋₃ alkyl-C₃₋₆ cycloalkyl, and -C₁₋₃ alkyl-3- to 6-membered heterocycloalkyl, and the C₃₋₆ cycloalkyl, 3-to 6-membered heterocycloalkyl, -C₁₋₃ alkyl-C₁₋₃ alkoxy, -C₁₋₃ alkyl-C₃₋₆ cycloalkyl, and - C₁₋₃ alkyl-3- to 6-membered heterocycloalkyl are each independently and optionally substituted by 1, 2, or 3 Rₐ groups, and other variables are as defined in the present disclosure.

In some embodiments of the present disclosure, the R₁ is selected from fluorine, chlorine, CH₃, CH₂CH₃, CH₂CH₂CH₃, CH(CH₃)₂, 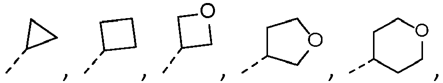 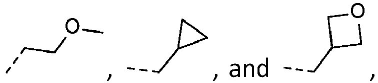 and the CH₃, CH₂CH₃, CH₂CH₂CH₃, CH(CH₃)₂, 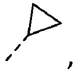 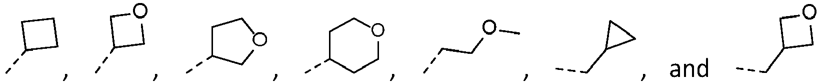 are each independently and optionally substituted by 1, 2, or 3 Rₐ groups, and other variables are as defined in the present disclosure.

In some embodiments of the present disclosure, the R₁ is selected from CH₃, CF₃, CH₂CH₃, CH₂CHF₂, CH₂CF₃, 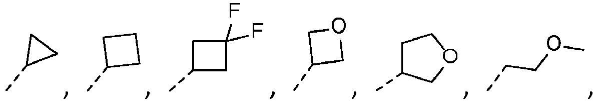 and 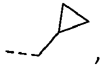 and other variables are as defined in the present disclosure.

In some embodiments of the present disclosure, the R₁ is selected from 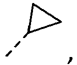 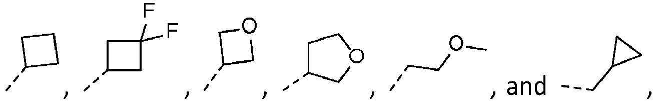 and other variables are as defined in the present disclosure.

In some embodiments of the present disclosure, the R₁ is selected from C₁₋₅ alkyl, the R₂ is selected from H and C₁₋₅ alkyl, the R₃ is selected from C₁₋₅ alkyl, and other variables are as defined in the present disclosure.

In some embodiments of the present disclosure, the R₁ is selected from CH₂CH₃, the R₂ is selected from H and CH₃, the R₃ is selected from CHF₂ and CH₃, and other variables are as defined in the present disclosure.

In some embodiments of the present disclosure, the R₂ is selected from H and CH₃, the CH₃ is optionally substituted by 1, 2, or 3 R_{b} groups, and other variables are as defined in the present disclosure.

In some embodiments of the present disclosure, the R₂ is selected from H, CH₃, and CHF₂, and other variables are as defined in the present disclosure.

In some embodiments of the present disclosure, the R₃ is selected from H and CH₃, and other variables are as defined in the present disclosure.

In some embodiments of the present disclosure, the R₄ is selected from H and CH₃, and other variables are as defined in the present disclosure.

In some embodiments of the present disclosure, the L is selected from the single bond and O, and other variables are as defined in the present disclosure.

In some embodiments of the present disclosure, the structural moiety 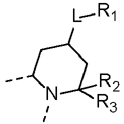 is selected from 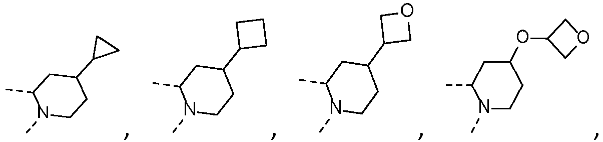 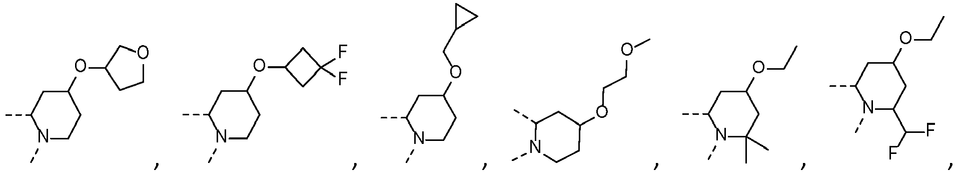 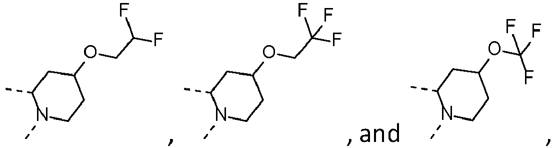 and other variables are as defined in the present disclosure.

In some embodiments of the present disclosure, the structural moiety 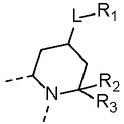 is selected from 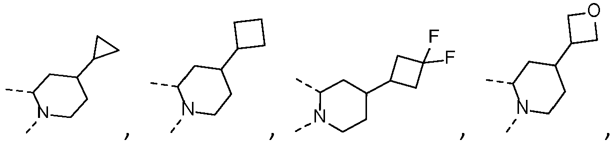 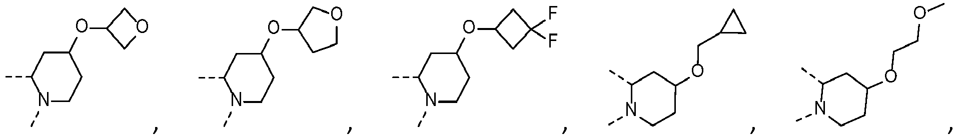 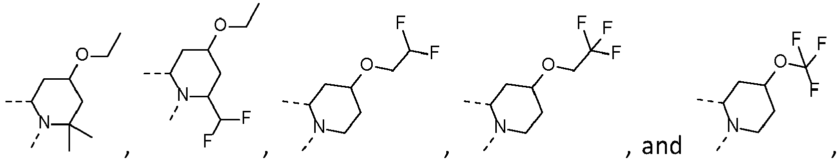 and other variables are as defined in the present disclosure.

In some embodiments of the present disclosure, the structural moiety 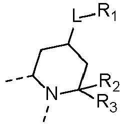 is selected from 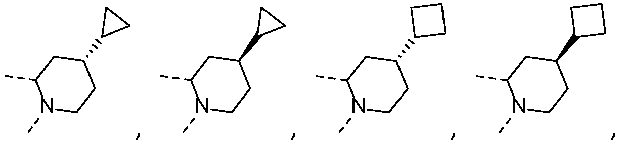 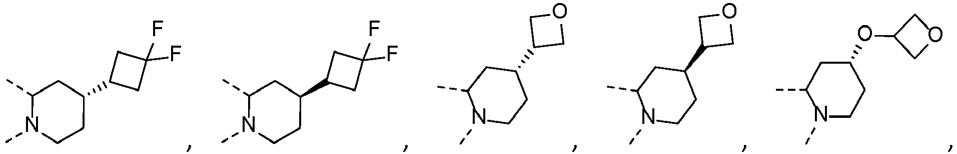 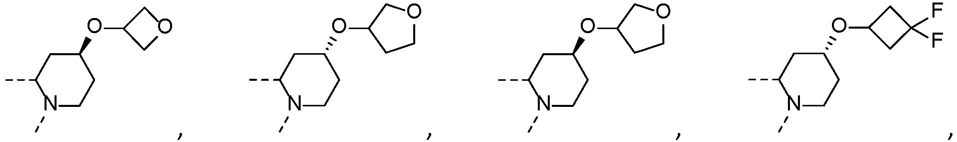 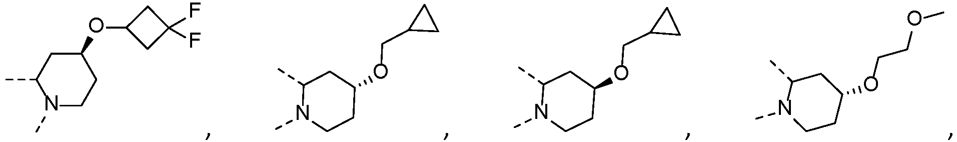 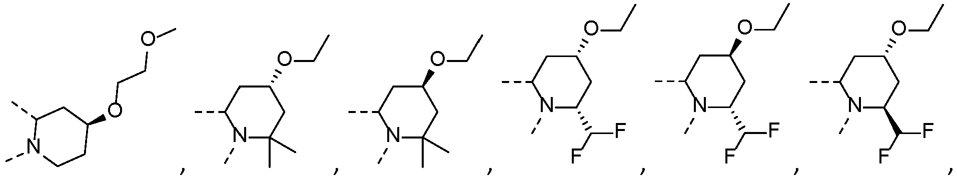 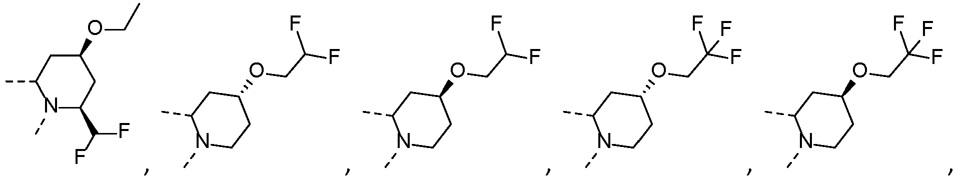 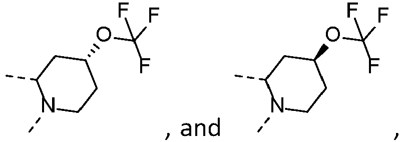 and other variables are as defined in the present disclosure.

The present disclosure provides a compound represented by formula (I) or a pharmaceutically acceptable salt thereof, 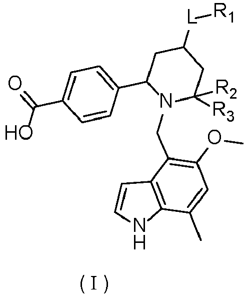 wherein
L is selected from a single bond, NR₄, and O;
R₁ is selected from fluorine, chlorine, C₁₋₅ alkyl, C₃₋₆ cycloalkyl, 3- to 6-membered heterocycloalkyl, -C₁₋₃ alkyl-C₁₋₃ alkoxy, -C₁₋₃ alkyl-C₃₋₆ cycloalkyl, and -C₁₋₃ alkyl-3- to 6-membered heterocycloalkyl, and the C₁₋₅ alkyl, C₃₋₆ cycloalkyl, 3- to 6-membered heterocycloalkyl, -C₁₋₃ alkyl-C₁₋₃ alkoxy, -C₁₋₃ alkyl-C₃₋₆ cycloalkyl, and -C₁₋₃ alkyl-3- to 6-membered heterocycloalkyl are each independently and optionally substituted by 1, 2, or 3 Rₐ groups;
R₂, R₃, and R₄ are each independently selected from H and C₁₋₅ alkyl, and the C₁₋₅ alkyl is optionally substituted by 1, 2, or 3 R_{b} groups;
each Rₐ and R_{b} are independently selected from H, F, Cl, Br, and I;
provided that R₂ and R₃ are not simultaneously selected from H when R₁ is selected from C₁₋₅ alkyl.

There are still some embodiments of the present disclosure which are obtained by any combination of the above variables.

The present disclosure also provides a compound represented by the following formulas or a pharmaceutically acceptable salt thereof:

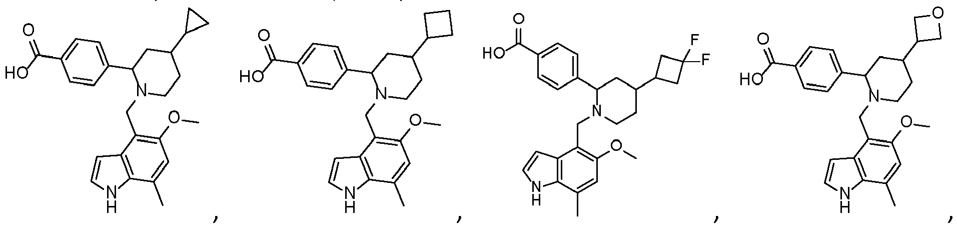

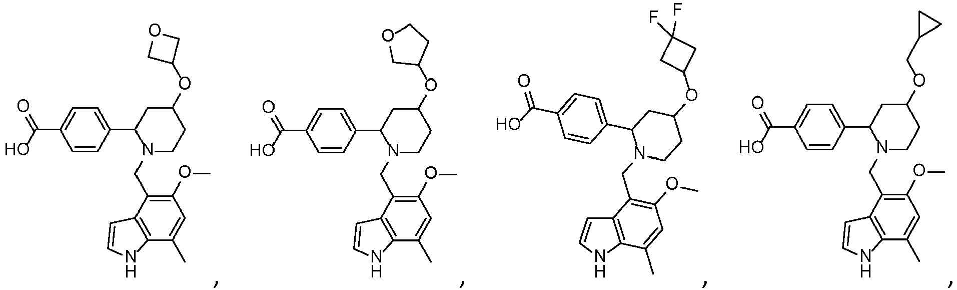

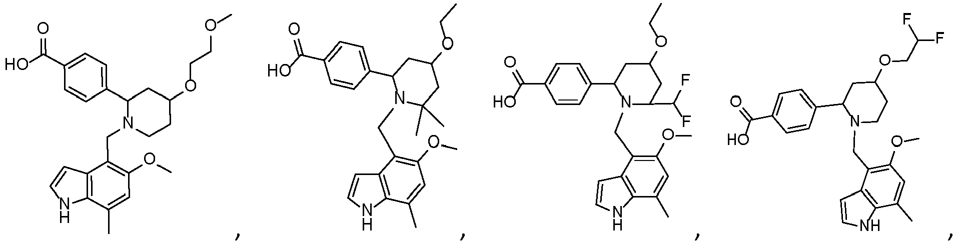

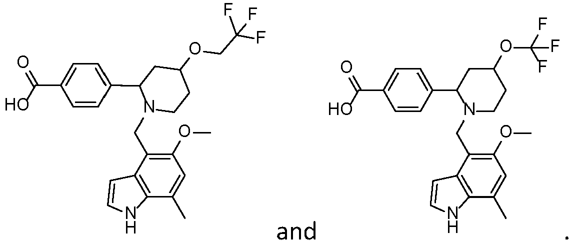

In some embodiments of the present disclosure, the compound or the pharmaceutically acceptable salt thereof is selected from:

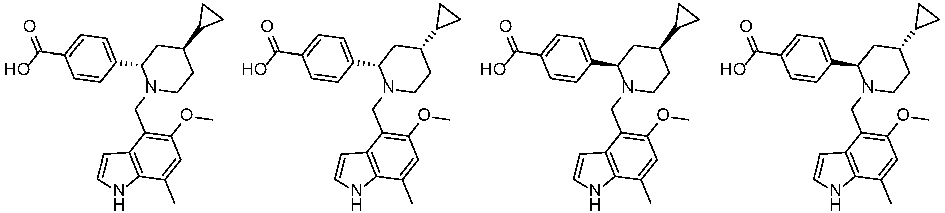

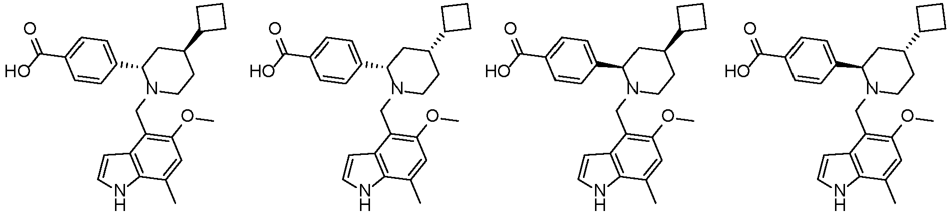

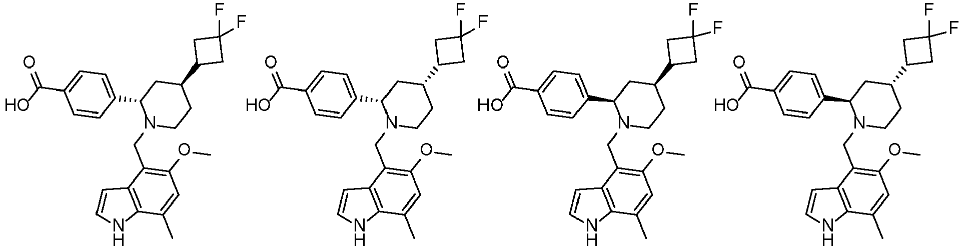

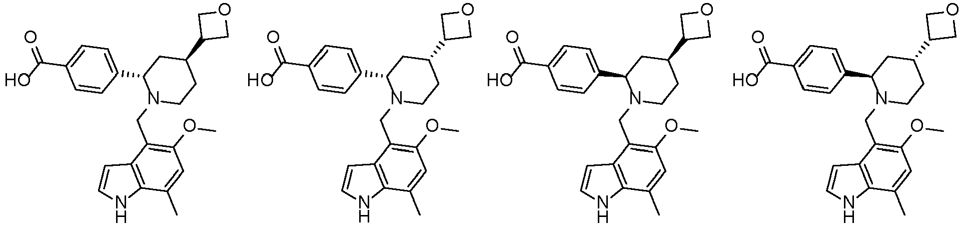

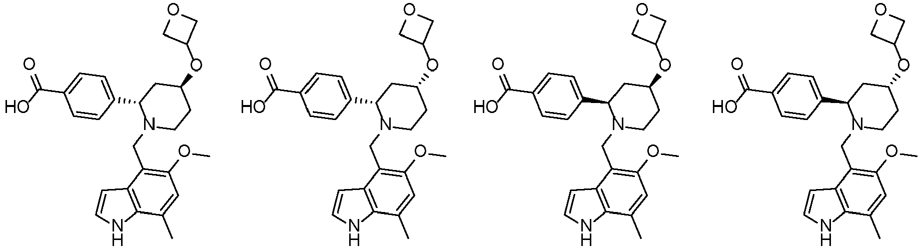

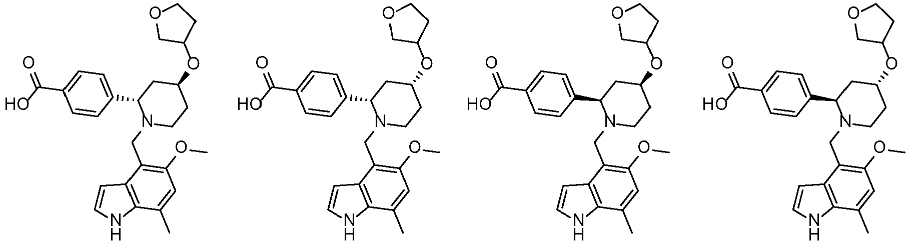

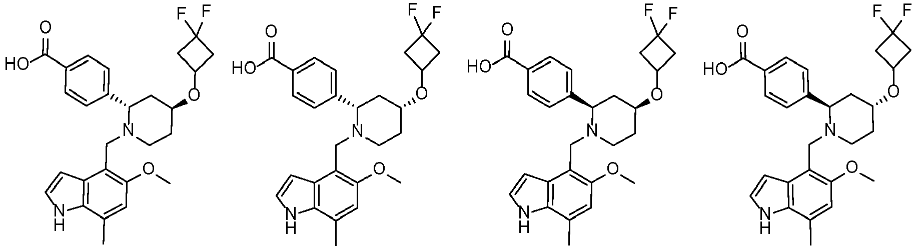

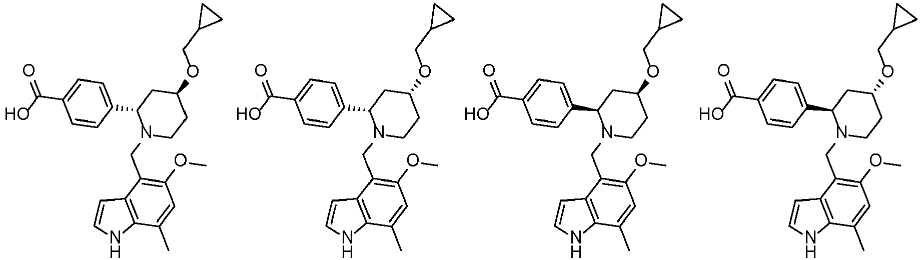

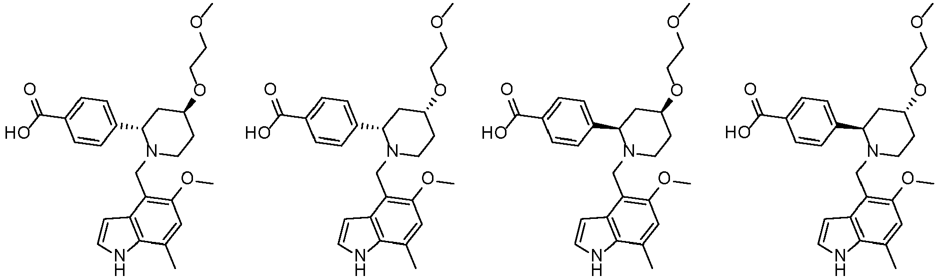

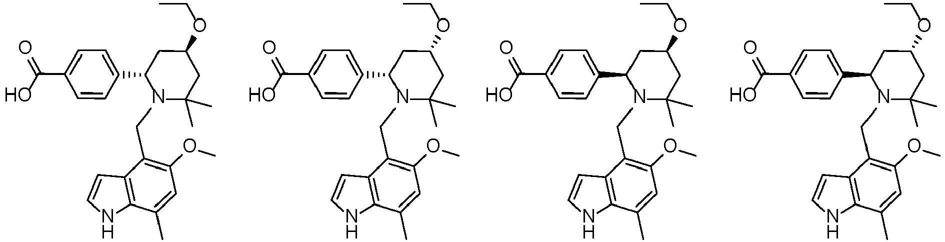

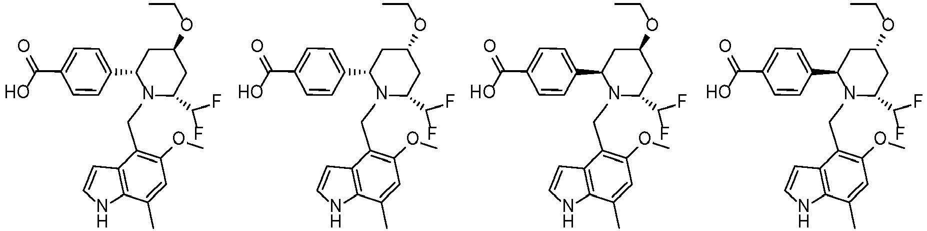

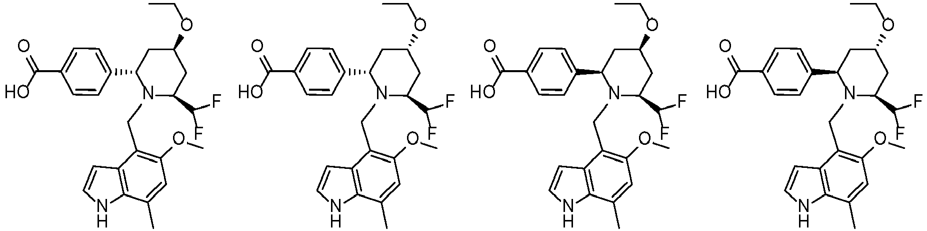

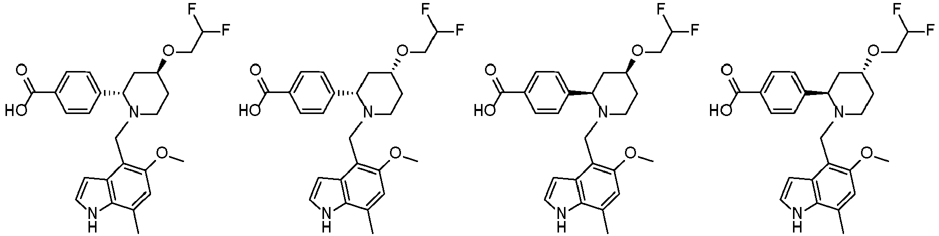

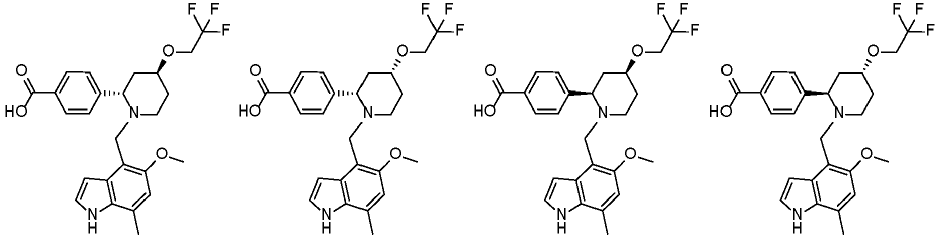

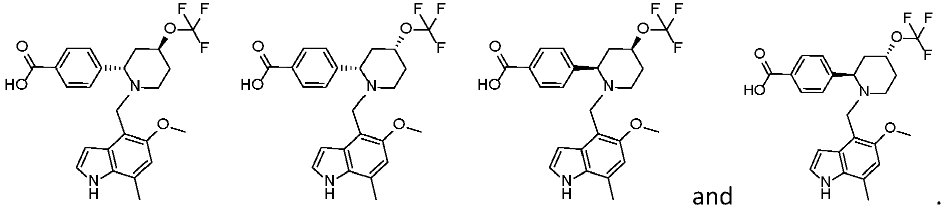

The present disclosure also provides a use of the compound or the pharmaceutically acceptable salt thereof in the manufacture of a medicament related to complement factor B.

The present disclosure also provides the following test method:

### Method 1. Drug efficacy test for passive Heymann nephritis (PHN)

1.1 Experimental objective: To investigate the ability of the compounds of the present disclosure to improve renal function of Sheep Anti-Rat Fx1A Serum induced Heymann nephritis in rats, including the evaluation of reducing proteinuria level and improving renal tissue damage.
1.2 Experimental animals: male SD rats, 7 to 10 weeks old, weighing 200 to 300 g
1.3 Experimental procedure:
   1.3.1 Modeling: Urine is collected from rats on D-2 before administration. On D1, the first day of the experiment, animals in the control group (group 1) are administered 5 mL/kg of Sheep Non-Immune serum via a single tail vein injection; animals in the model and administration groups (groups 2 to 6) are administered 5 mL/kg of Sheep Anti-Rat Fx1A Serum via a single tail vein injection.
   1.3.2 Administration:
      The rats are treated by intragastric administration twice a day with an interval of 8 hours and a volume of 10 mL/kg. On D1, 1 hour before modeling, animals in groups 1 and 2 are administered blank vehicle 20% PEG 400/10% Solutol/70% water; animals in group 3 are administered **LNP023** (60 mpk); animals in groups 4 to 6 are administered different concentrations of compound **4B** (5 mpk, 20 mpk, and 60 mpk); 8 hours after the first administration, each group is administered again with the same dose and volume as the first administration. From D2 to D14, animals in each group are administered different compounds or vehicles for 14 consecutive days (including the first day) according to the dose, volume, administration method, and frequency on D1.
   1.3.3 Sample collection:
      (1) Urine collection: Urine samples are collected from rats on D-2 before administration, and 2 to 4 hours and 4 to 6 hours after the first administration on D4, D6, D8, D11, and D14 after administration, transferred to EP tubes and stored in a refrigerator at -80°C to -60°C for the detection of urine protein and creatinine in rats.
      (2) Kidney tissue collection: All animals are euthanized with CO₂ inhalation anesthesia on D15 after administration, and bilateral kidneys are collected. The left kidney is cut transversely, and the right kidney is cut longitudinally. Half of the transversely cut kidney (left side) and half of the longitudinally cut kidney (right side) are fixed in formalin (placed in the same EP tube). The remaining half of the transversely cut kidney (left side) and the remaining half of the longitudinally cut kidney (right side) are embedded in OCT with the cut side facing downward (placed in the same embedding box). The samples are stored in a refrigerator at -80°C to -60°C as soon as possible after embedding for histopathological scoring.
   1.3.4 Sample analysis: Urine protein and creatinine data are read by the analytical instrument HITACHI LST008 AS(P). Renal glomerulopathy (characterized by enlarged glomeruli, thickened basement membrane and Bowman's capsule, and enlarged/rounded podocytes) is scored by histopathological analysis, tubular degeneration score is determined by histopathological analysis with IHC or IF staining, and glomerular C3 deposition is scored.

### Technical effect

The compounds of the present disclosure have significant inhibitory activity on the activation of the alternative pathway in human serum, can significantly inhibit LPS-stimulated complement activation, reduce urine protein level, and improve renal function; PK results show that the compounds of the present disclosure exhibit a long half-life and a high drug exposure, good in vivo pharmacokinetic properties, and good druggability.

### Definition and description

Unless otherwise specified, the following terms and phrases when used herein have the following meanings. A specific term or phrase should not be considered indefinite or unclear in the absence of a particular definition, but should be understood in the ordinary sense. When a trading name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the compound into contact with a sufficient amount of base in a pure solution or a suitable inert solvent. The pharmaceutically acceptable base addition salt includes a salt of sodium, potassium, calcium, ammonium, organic amine or magnesium, or similar salts. When the compound of the present disclosure contains a relatively basic functional group, an acid addition salt can be obtained by bringing the compound into contact with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid, and the like; and salts of amino acid (such as arginine and the like), and a salt of an organic acid such as glucuronic acid and the like. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including *cis* and *trans* isomers, (-)- and (+)-enantiomers, (*R*)- and (*S*)-enantiomers, diastereomers isomers, (*D*)-isomers, (*L*)-isomers, and racemic and other mixtures thereof, such as enantiomers or diastereomeric enriched mixtures, all of which are within the scope of the present disclosure. Additional asymmetric carbon atoms may be present in substituents such as alkyl. All these isomers and their mixtures are included within the scope of the present disclosure.

Unless otherwise specified, the term "enantiomer" or "optical isomer" refers to stereoisomers that are mirror images of each other.

Unless otherwise specified, the term "*cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and "(±)" refers to racemic.

Unless otherwise specified, the absolute configuration of a stereogenic center is represented by a wedged solid bond ( 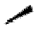 ) and a wedged dashed bond ( 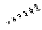 ), and the relative configuration of a stereogenic center is represented by a straight solid bond ( 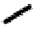 ) and a straight dashed bond ( 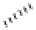 ), a wave line ( 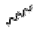 ) is used to represent a wedged solid bond ( 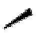 ) or a wedged dashed bond ( 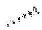 ), or the wave line ( 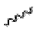 ) is used to represent a straight solid bond ( 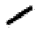 ) and a straight dashed bond ( 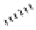 ).

Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) includes interconversion through proton migration, such as keto-enol isomerization and imine-enamine isomerization. Valence tautomer includes some recombination of bonding electrons for mutual transformation. A specific example of keto-enol tautomerization is the tautomerism between two tautomers of pentane-2,4-dione and 4-hydroxypent-3-en-2-one.

Unless otherwise specified, the terms "enriched in one isomer", "enriched in isomers", "enriched in one enantiomer" or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

The compound of the present disclosure may contain an unnatural proportion of atomic isotope at one or more than one atom(s) that constitute the compound. For example, the compound can be radiolabeled with a radioactive isotope, such as tritium (³H), iodine-125 (¹²⁵I) or C-14 (¹⁴C). For another example, deuterated drugs can be formed by replacing hydrogen with heavy hydrogen, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

The term "optional" or "optionally" means that the subsequent event or condition may occur but not requisite, that the term includes the instance in which the event or condition occurs and the instance in which the event or condition does not occur.

The term "substituted" means one or more than one hydrogen atom(s) on a specific atom are substituted with the substituent, including deuterium and hydrogen variables, as long as the valence of the specific atom is normal and the substituted compound is stable. When the substituent is an oxygen (i.e., =O), it means two hydrogen atoms are substituted. Positions on an aromatic ring cannot be substituted with a ketone. The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted with 0 to 2 R, the group can be optionally substituted with up to two R, wherein the definition of R at each occurrence is independent. Moreover, a combination of the substituent and/or the variant thereof is allowed only when the combination results in a stable compound.

When the number of a linking group is 0, such as -(CRR)₀-, it means that the linking group is a single bond.

When one of the variables is selected from a single bond, it means that the two groups linked by the single bond are connected directly. For example, when L in A-L-Z represents a single bond, the structure of A-L-Z is actually A-Z.

When the enumerative linking group does not indicate the direction for linking, the direction for linking is arbitrary, for example, the linking group L contained in 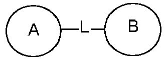 is -M-W-, then -M-W- can link ring A and ring B to form 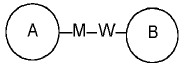 in the direction same as left-to-right reading order, and form 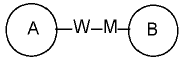 in the direction contrary to left-to-right reading order. A combination of the linking groups, substituents and/or variables thereof is allowed only when such combination can result in a stable compound.

Unless otherwise specified, when a group has one or more linkable sites, any one or more sites of the group can be linked to other groups through chemical bonds. When the linking site of the chemical bond is not positioned, and there is an H atom at the linkable site, then the number of H atoms at the site will decrease correspondingly with the number of the chemical bonds linking thereto so as to meet the corresponding valence. The chemical bond between the site and other groups can be represented by a straight solid bond 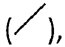 a straight dashed bond 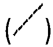 or a wavy line 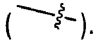 For example, the straight solid bond in -OCH₃ means that it is linked to other groups through the oxygen atom in the group; the straight dashed bond in 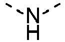 means that it is linked to other groups through the two ends of nitrogen atom in the group; the wave line in 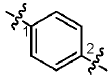 means that the phenyl group is linked to other groups through carbon atoms at position 1 and position 2; 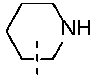 means that it can be linked to other groups through any linkable sites on the piperidinyl by one chemical bond, including at least four types of linkage, including 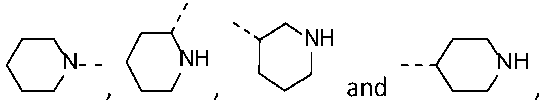 even though the H atom is drawn on the -N-, 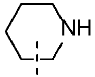 still includes the linkage of 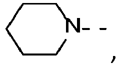 merely when one chemical bond was connected, the H of this site will be reduced by one to the corresponding monovalent piperidinyl.

Unless otherwise specified, the term "C₁₋₃ alkyl" by itself or in combination with other terms refers to a linear or branched saturated hydrocarbon group consisting of 1 to 3 carbon atoms. The C₁₋₃ alkyl includes C₁₋₂ and C₂₋₃ alkyl and the like; it can be monovalent (such as methyl), divalent (such as methylene) or multivalent (such as methine). Examples of C₁₋₃ alkyl include but are not limited to methyl (Me), ethyl (Et), propyl (including *n*-propyl and isopropyl) and the like.

Unless otherwise specified, the term "C₁₋₅ alkyl" by itself or in combination with other terms refers to a linear or branched saturated hydrocarbon group consisting of 1 to 5 carbon atoms. The C₁₋₅ alkyl includes C₁₋₄, C₁₋₃, C₁₋₂, C₂₋₅, C₂₋₄ and C₅ alkyl, etc.; it can be monovalent (such as methyl), divalent (such as methylene), or multivalent (such as methine). Examples of C₁₋₅ alkyl include, but are not limited to, methyl (Me), ethyl (Et), propyl (including *n*-propyl and isopropyl), butyl (including *n*-butyl, isobutyl, *s*-butyl, and *t*-butyl), pentyl (including *n*-pentyl, isopentyl, and neopentyl), etc.

Unless otherwise specified, the term "C₁₋₃ alkoxy" by itself or in combination with other terms refers to an alkyl consisting of 1 to 3 carbon atoms that are connected to the rest of the molecule through an oxygen atom. The C₁₋₃ alkoxy includes C₁₋₂, C₂₋₃, C₃, and C₂ alkoxy, etc. Examples of C₁₋₃ alkoxy include, but are not limited to, methoxy, ethoxy, propoxy (including *n*-propoxy and isopropoxy) and the like.

Unless otherwise specified, "C₃₋₆ cycloalkyl" by itself or in combination with other terms refers to a saturated monocyclic hydrocarbon group consisting of 3 to 6 carbon atoms, and the C₃₋₆ cycloalkyl includes C₃₋₅, C₄₋₅, and C₅₋₆ cycloalkyl, etc.; it can be monovalent, divalent, or multivalent. Examples of C₃₋₆ cycloalkyl include, but are not limited to, cyclopropyl, cyclobutyl, cyclopentyl, cyclohexyl, etc.

Unless otherwise specified, the term "3- to 6-membered heterocycloalkyl" by itself or in combination with other terms refers to a saturated cyclic group consisting of 3 to 6 ring atoms, wherein 1, 2, 3, or 4 ring atoms are heteroatoms independently selected from O, S, and N, and the rest are carbon atoms, wherein nitrogen atoms are optionally quaternized, and nitrogen and sulfur heteroatoms can be optionally oxidized (i.e., NO and S(O)ₚ, p is 1 or 2). In addition, with regard to the "3- to 6-membered heterocycloalkyl", a heteroatom may occupy the connection position of the heterocycloalkyl with the rest of the molecule. The 3- to 6-membered heterocycloalkyl includes 4- to 6-membered, 5- to 6-membered, 4-membered, 5-membered, and 6-membered heterocycloalkyl, etc. Examples of 3- to 6-membered heterocycloalkyl include, but are not limited to, azetidinyl, oxetanyl, thietanyl, pyrrolidinyl, pyrazolidinyl, imidazolidinyl, tetrahydrothienyl (including tetrahydrothiophen-2-yl and tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, and 3-piperidinyl, etc.), piperazinyl (including 1-piperazinyl and 2-piperazinyl, etc.), morpholinyl (including 3-morpholinyl and 4-morpholinyl, etc.), dioxinyl, dithianyl, isoxazolidinyl, isothiazolidinyl, 1,2-oxazinyl, 1,2-thiazinyl, or hexahydropyridazinyl, etc.

Unless otherwise specified, Cₙ₋ₙ₊ₘ or Cₙ-Cₙ₊ₘ includes any specific case of n to n+m carbons, for example, C₁₋₁₂ includes C₁, C₂, C₃, C₄, C₅, C₆, C₇, C₈, C₉, C₁₀, C₁₁, and C₁₂, and any range from n to n+m is also included, for example C₁₋₁₂ includes C₁₋₃, C₁₋₆, C₁₋₉, C₃₋₆, C₃₋₉, C₃₋₁₂, C₆₋₉, C₆₋₁₂, C₉₋₁₂ and the like; similarly, n-membered to n+m-membered means that the number of atoms on the ring is from n to n+m, for example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring, and any range from n to n+m is also included, for example, 3- to 12-membered ring includes 3- to 6-membered ring, 3- to 9-membered ring, 5- to 6-membered ring, 5- to 7-membered ring, 6- to 7-membered ring, 6- to 8-membered ring, and 6- to 10-membered ring, etc.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure.

The solvent used in the present disclosure is commercially available.

The following abbreviations are used in the present disclosure: aq refers to water; eq refers to equivalent or equivalent weight; DCM refers to dichloromethane; PE refers to petroleum ether; DMSO refers to dimethyl sulfoxide; EtOAc refers to ethyl acetate; EtOH refers to ethanol; MeOH refers to methanol; DMF refers to N,N-dimethylformamide; Cbz refers to benzyloxycarbonyl, which is a protective group for amino; BOC refers to tert-butoxycarbonyl, which is a protective group for amino; r.t. refers to room temperature; RT refers to retention time; O/N refers to overnight; THF refers to tetrahydrofuran; Boc₂O refers to di-tert-butyl dicarbonate; TFA refers to trifluoroacetic acid; HCl refers to hydrochloric acid; DIPEA refers to diisopropylethylamine; TEA refers to triethylamine; NBS refers to N-bromosuccinimide; iPrOH refers to 2-propanol; mp refers to melting point; Pd(PPh₃)₄ refers to tetrakis(triphenylphosphine)palladium; Pd(dppf)Cl₂·CH₂Cl₂ refers to [1,1'-bis(diphenylphosphino)ferrocene]palladium(II)dichloride dichloromethane complex; Pd(dppf)Cl₂ refers to [1,1'-bis(diphenylphosphino)ferrocene]palladium(II)dichloride; PPA refers to polyphosphoric acid; NMP refers to N-methylpyrrolidone; TBSCI refers to tert-butyldimethylsilyl chloride; n-BuLi refers to n-butyllithium; TBAF refers to tetrabutylammonium fluoride; psi refers to pounds per square inch; CO₂ refers to carbon dioxide; DEA refers to diethylamine; PEG300 refers to polyethylene glycol 300; Cremphor EL refers to polyoxyethylene castor oil; PBS refers to phosphate buffered saline.

The structure of the compounds of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), diffraction intensity data are collected from the cultured single crystal using a Bruker D8 venture diffractometer with CuKα radiation as the light source and scanning mode: φ/ω scan, and after collecting the relevant data, the crystal structure is further analyzed by direct method (Shelxs97), so that the absolute configuration can be confirmed.

### BRIEF DESCRIPTION OF THE DRAWINGS

Figure 1 shows the experimental results of in vivo PD model of LPS-induced complement activation in mice;
Figure 2 shows the experimental results of in vivo pharmacodynamic model of passive Heymann nephritis in rats.

### DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific examples have also been disclosed; for one skilled in the art, it is obvious to make various modifications and improvements to the examples of the present disclosure without departing from the spirit and scope of the present disclosure.

### Reference example 1: Preparation of intermediates M-2 and M-1

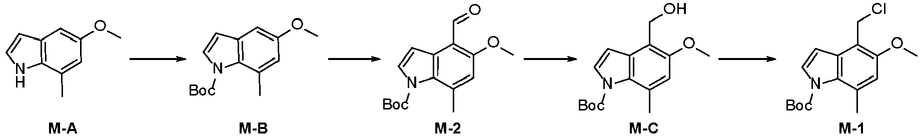

### Step 1

To a solution of compound **M-A** (5.00 g) in acetonitrile (30.0 mL) was added 4-dimethylaminopyridine (3.79 g) and di-tert-butyl dicarbonate (8.12 g) at 20°C. The reaction mixture was stirred at 20°C for 1 hour, diluted with ethyl acetate (100.0 mL), washed with 1 N hydrochloric acid (30.0 mL) and saturated brine (50.0 mL × 2) successively, dried over anhydrous sodium sulfate, filtered, and concentrated to obtain compound **M-B.** ¹H NMR (400 MHz, CDCl₃) δ 7.52 (d, J =4.0 Hz, 1H), 6.87 (d, J =2.4 Hz, 1H), 6.75 (d, J =2.4 Hz, 1H), 6.48 (d, J =4.0 Hz, 1H), 3.85 (s, 3H), 2.64 (s, 3H), 1.65 (s, 9H).

### Step 2

To a solution of *N*-methylformanilide (5.59 g) in dichloromethane (50.0 mL) was added oxalyl chloride (5.25 g) at 20°C. The reaction mixture was stirred at 20°C for 14 hours under nitrogen atmosphere. The reaction mixture was transferred to a constant pressure dropping funnel, slowly added dropwise to a solution of compound **M-B** (9.00 g) in dichloromethane (50.0 mL) at -14°C, and stirred at -14°C for 3 hours under nitrogen atmosphere. The reaction mixture was added with saturated sodium bicarbonate (50.0 mL) to quench, and washed with saturated brine (50 mL × 2). The organic phase was dried over anhydrous sodium sulfate, filtered, and the filtrate was concentrated under reduced pressure. The residue was washed with ethanol (20.0 mL), filtered, and the filter cake was dried to obtain compound **M-2.** ¹H NMR (400 MHz, CDCl₃) δ 10.62(s, 1H), 7.62 (d, J = 3.6 Hz, 1H), 7.47 (d, J = 3.6 Hz, 1H), 6.73 (s, 1H), 3.95 (s, 3H), 2.68 (s, 3H), 1.63 (s, 9H); LC-MS: m/z = 290.1 [M+H]⁺.

### Step 3

To a solution of compound **M-2** (3.00 g, 10.37 mmol, 1 eq) in methanol (10.0 mL) was added sodium borohydride (980.7 mg) at 0°C, and the reaction mixture was stirred at 0°C for 2 hours. After the reaction was completed, the reaction mixture was added with saturated ammonium chloride solution (200.0 mL) at 0°C. The reaction mixture was then diluted with water (100.0 mL), extracted with ethyl acetate (20.0 mL × 2), and washed with water (50.0 mL) and saturated brine (50.0 mL). The organic phases were combined, dried over anhydrous sodium sulfate, filtered, and the filtrate was concentrated under reduced pressure. The residue was purified by silica gel column chromatography (ethyl acetate: petroleum ether = 2.2:1) to obtain compound **M-C.** ¹H NMR (400 MHz, CDCl₃) δ 7.53 (d, J = 3.60 Hz, 1H), 6.74 (s, 1H), 6.61 (d, J = 3.60 Hz, 1H), 4.89 (s, 2H), 3.90 (s, 3H), 2.63 (s, 3H), 1.62 (s, 9H).

### Step 4

To a solution of compound **M-C** (1.60 g) in methanol (1.2 mL) and dichloromethane (20.0 mL) was added (chloromethylene)dimethylammonium chloride (1.41 g) at 20°C, and the reaction mixture was stirred at the same temperature for 1 hour. The reaction mixture was cooled to 0°C, added with saturated sodium bicarbonate solution (20.0 mL) to quench, and extracted with ethyl acetate (30 mL × 3). The organic phases were combined, washed with saturated brine (20.0 mL × 2), and dried over anhydrous sodium sulfate. The mixture was filtered, and the filtrate was concentrated under reduced pressure to obtain compound **M-1.**

### Example 1: Preparation of compounds 1A, 1B, 1C, and 1D

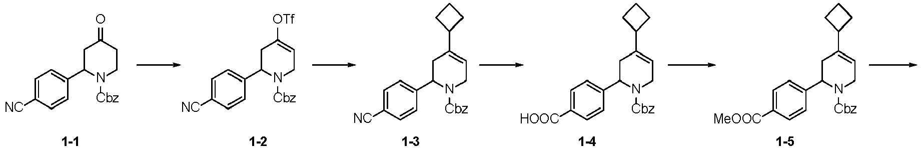

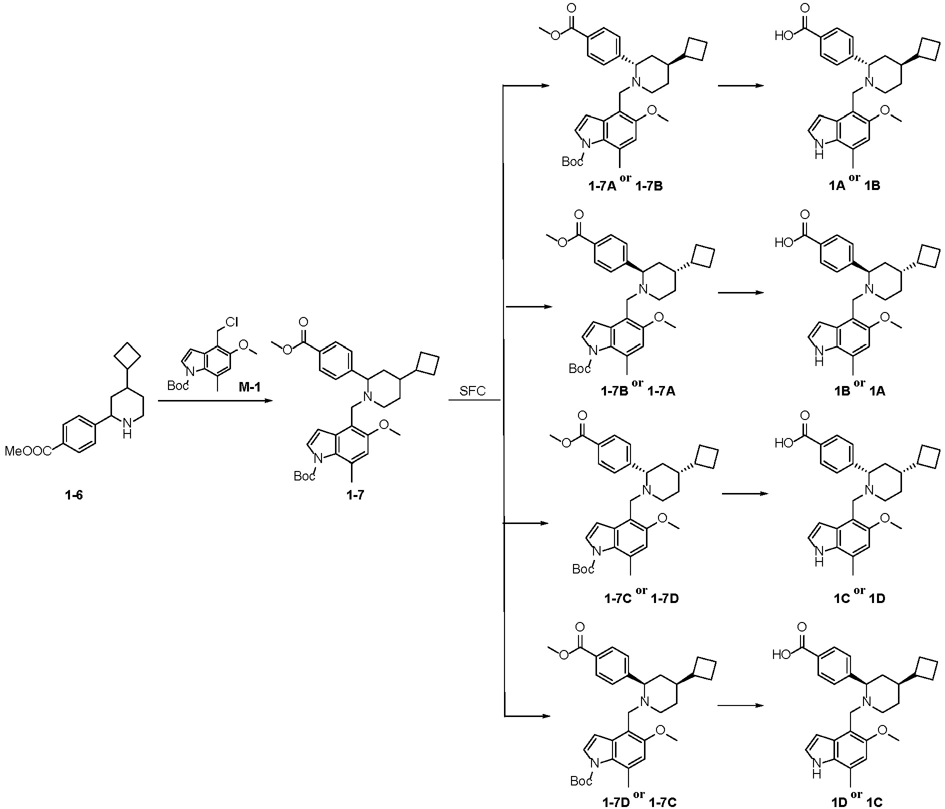

### Step 1

Compound **1-1** (3.20 g) was dissolved in tetrahydrofuran (30 mL), and the reaction mixture was placed at -78°C, added with lithium bis(trimethylsilyl)amide (1 M, 10.5 mL) under nitrogen atmosphere, stirred at the same temperature for 1 hour, then warmed to 0°C, and added with compound 1,1,1-trifluoro-*N*-phenyl-*N-*((trifluoromethyl)sulfonyl)methanesulfonamide (5.10 g). The reaction mixture was warmed to 30°C, and stirred for 12 hours. The reaction mixture was added with water (15 mL) to quench, and extracted with ethyl acetate (40 mL × 3). The organic phase was washed with saturated brine (40 mL × 1), dried over anhydrous sodium sulfate, filtered, and concentrated. The residue was purified by silica gel column chromatography (petroleum ether: ethyl acetate = 1:0 to 2:1) to obtain compound **1-2.** ¹H NMR (400 MHz, CDCl₃) δ 7.56-7.58 (m, 2H), 7.28-7.38 (m, 6H), 7.17-7.23 (m, 1H), 5.89 (br s, 1H), 5.01-5.19 (m, 3H), 4.22 (br s, 1H), 2.92-2.99 (m, 1H), 2.58-2.73 (m, 1H), 2.20-2.38 (m, 1H); LC-MS: m/z =467.0 [M+H]⁺.

### Step 2

To a solution of compound **1-2** (0.50 g) and cyclobutylboronic acid (139.3 mg) in toluene (10 mL) was added [1,1-bis(diphenylphosphino)ferrocene]palladium(II)dichloride dichloromethane complex (175.1 mg) and cesium carbonate (1.05 g) at 20°C. The reaction mixture was heated to 110°C, and stirred for 14 hours under nitrogen atmosphere. The reaction mixture was cooled to room temperature, added with ice water (20 mL) to quench, and extracted with ethyl acetate (25 mL × 3). The organic phase was dried over anhydrous sodium sulfate, filtered, and the filtrate was concentrated under reduced pressure. The residue was purified by silica gel column chromatography (ethyl acetate: petroleum ether = 1:10) to obtain compound **1-3.** ¹H NMR (400 MHz, CDCl₃) δ ppm 7.30-7.67 (m, 9H), 5.37-5.78 (m, 2H), 5.18-5.24 (m, 1H), 5.06-5.17 (m, 1H), 4.17-4.38 (m, 1H), 2.86-3.01 (m, 2H), 2.16-2.28 (m, 1H), 2.08-2.17 (m, 2H), 1.88-2.02 (m, 4H), 1.71-1.82 (m, 1H); LC-MS: *m*/*z* = 373.1 [M+H]⁺.

### Step 3

To a mixed solution of compound **1-3** (0.34 g) in isopropanol (2 mL), dioxane (2 mL), and water (4 mL) was added barium hydroxide (1.16 g), and the reaction mixture was heated to 100°C and stirred for 20 hours. The reaction mixture was cooled to room temperature, added with 50% potassium bisulfate aqueous solution to adjust the pH < 7, and extracted with ethyl acetate (40 mL × 3). The organic phases were combined, washed once with water (30 mL) and once with saturated brine (25 mL) successively, and dried over anhydrous sodium sulfate. The reaction mixture was filtered, and the filtrate was concentrated under reduced pressure to obtain compound **1-4.** LC-MS: *m*/*z* = 392.1 [M+H]⁺.

### Step 4

To a mixed solution of compound **1-4** (0.36 g) in methanol (1.2 mL) and toluene (3.6 mL) was added (trimethylsilyl)diazomethane (2 M, 919 µL) at 20°C, and the reaction mixture was stirred at 20°C for 1 hour. The reaction mixture was cooled to 0°C, added with acetic acid to quench, diluted with water (40 mL), and extracted with ethyl acetate (35 mL × 3). The organic phases were combined, washed with saturated sodium bicarbonate aqueous solution (25 mL), water (25 mL), and saturated brine (25 mL) successively, dried over anhydrous sodium sulfate, and filtered. The filtrate was concentrated under reduced pressure, and the residue was purified by silica gel column chromatography (ethyl acetate: petroleum ether = 1:3) to obtain compound **1-5.** LC-MS: *m*/*z* = 406.1 [M+H]⁺.

### Step 5

To a solution of compound **1-5** (0.24 g) in ethanol (4 mL) was added wet palladium on carbon (10%, 0.20 g) and a solution of hydrogen chloride in dioxane (4 M, 0.04 mL) at 20°C, and the reaction mixture was stirred at 20°C for 1 hour under hydrogen atmosphere (15 psi). The reaction mixture was filtered, and the filtrate was concentrated under reduced pressure to obtain compound **1-6.** LC-MS: *m*/*z* = 274.1 [M+H]⁺.

### Step 6

To a solution of compound **1-6** (0.55 g) in *N,N*-dimethylformamide (15 mL) was added compound **M-1** (1.01 g), cesium carbonate (1.64 g), and potassium iodide (334.0 mg) at 20°C. The reaction mixture was stirred at 20°C for 15 hours, slowly poured into water (80 mL), and extracted with ethyl acetate (90 mL × 3). The organic phases were combined, dried over anhydrous sodium sulfate, and filtered. The filtrate was concentrated under reduced pressure, and the residue was purified by silica gel column chromatography (ethyl acetate: petroleum ether = 1:1). The resulting crude product was then purified by preparative high performance liquid chromatography (HPLC) (chromatographic column: Phenomenex Gemini-NX 80 × 30 mm × 3 µm; mobile phase: [water (10 mM ammonium bicarbonate)-acetonitrile]; gradient: acetonitrile%: 57% to 87%) to obtain compound **1-7,** and chirally separated (chromatographic column: DAICEL CHIRALPAK AD (250 mm × 30 mm, 10 µm; mobile phase: A: carbon dioxide, B: [0.1% ammonia water-isopropanol]; gradient: B%: 35% to 35%) to obtain compound **1-7A,** compound **1-7B,** compound **1-7C,** and compound **1-7D.**

SFC analysis and detection method: chromatographic column: Chiralpak AD-3 150 mm × 4.6 mm I.D., 3 µm, mobile phase: A: carbon dioxide, B: isopropanol (0.05% diethylamine), gradient: B%: flowing from 5% to 40% for 5 min, holding at 40% for 5 min, holding at 5% for 2.5 min, flow rate: 2.5 mL/min. Retention time of compound **1-7A:** 4.238 min, ee: 100%; retention time of compound **1-7B:** 4.946 min, ee: 100%; retention time of compound **1-7C:** 5.530 min, ee: 99.4%; retention time of compound **1-7D:** 5.954 min, ee: 100%.

It was identified by two-dimensional NMR that the carbon-hydrogen bond by which the piperidine ring is linked to 4-membered cyclobutyl in compound **1-7C** has a NOE correlation with the carbon-hydrogen bond by which the piperidine ring is linked to benzoic acid in compound **1-7C,** and compound **1-7C** is in the cis configuration. The carbon-hydrogen bond by which the piperidine ring is linked to 4-membered cyclobutyl in compound **1-7D** has a NOE correlation with the carbon-hydrogen bond by which the piperidine ring is linked to benzoic acid in compound **1-7D,** and compound **1-7D** is in the cis configuration. LC-MS: *m*/*z* = 547.2 [M+H]⁺.

### Step 7

To a mixed solution of compound **1-7A** (15.0 mg) in methanol (1 mL) and tetrahydrofuran (0.5 mL) was added lithium hydroxide monohydrate (3.5 mg), and the reaction mixture was stirred at 50°C for 18 hours. The reaction mixture was concentrated under reduced pressure, and the residue was purified by preparative HPLC (chromatographic column: Phenomenex Gemini-NX 80 × 30 mm × 3 µm; mobile phase: [water (10 mM ammonium bicarbonate)-acetonitrile]; acetonitrile%: 16% to 86%) to obtain compound **1A.** LC-MS: *m*/*z* = 433.2 [M+H]⁺.

To a mixed solution of compound **1-7B** (20.0 mg) in methanol (1 mL) and tetrahydrofuran (0.5 mL) was added lithium hydroxide monohydrate (4.6 mg), and the reaction mixture was stirred at 50°C for 18 hours. The reaction mixture was concentrated under reduced pressure, and the residue was purified by preparative HPLC (chromatographic column: Phenomenex Gemini-NX 80 × 30 mm × 3 µm; mobile phase: [water (10 mM ammonium bicarbonate)-acetonitrile]; acetonitrile%: 16% to 86%) to obtain compound **1B.** LC-MS: *m*/*z* = 433.3 [M+H]⁺.

To a mixed solution of compound **1-7C** (0.10 g) in methanol (1 mL) and tetrahydrofuran (0.5 mL) was added lithium hydroxide monohydrate (23.0 mg), and the reaction mixture was stirred at 50°C for 15 hours. The reaction mixture was concentrated under reduced pressure, and the residue was purified by preparative HPLC (chromatographic column: Phenomenex Gemini-NX 80 × 30 mm × 3 µm; mobile phase: [water (10 mM ammonium bicarbonate)-acetonitrile]; acetonitrile%: 16% to 86%) to obtain compound **1C.** ¹H NMR (400 MHz, DMSO-*d*₆) δ ppm 10.80 (s, 1H), 7.95 (d, *J* = 8.80 Hz, 2H), 7.64 (d, *J* = 7.60 Hz, 2H), 7.24 (t, *J* = 2.80 Hz, 1H), 6.65 (s, 1H), 6.45 (t, *J* = 2.40 Hz, 1H), 3.70 (s, 3H), 3.54 (d, *J* = 12.00 Hz, 1H), 3.10-3.30 (m, 2H), 2.78-2.81 (m, 1H), 2.50-2.55 (m, 1H), 2.41 (s, 3H), 1.56 - 2.01 (m, 8H), 1.48-1.51 (m, 1H), 1.34 (m, 1H), 1.02-1.11 (m, 1H), 0.81 - 0.93 (m, 1H); LC-MS: *m*/*z* = 433.3 [M+H]⁺.

To a mixed solution of compound **1-7D** (0.10 g) in methanol (1 mL) and tetrahydrofuran (0.5 mL) was added lithium hydroxide monohydrate (23.0 mg), and the reaction mixture was stirred at 50°C for 15 hours. The reaction mixture was concentrated under reduced pressure, and the residue was purified by preparative HPLC (chromatographic column: Phenomenex Gemini-NX 80 × 30 mm × 3 µm; mobile phase: [water (10 mM ammonium bicarbonate)-acetonitrile]; acetonitrile%: 16% to 86%) to obtain compound **1D.** ¹H NMR (400 MHz, DMSO-*d*₆) δ ppm 10.80 (s, 1H), 7.95 (d, *J* = 8.40 Hz, 2H), 7.64 (d, *J* = 7.60 Hz, 2H), 7.24 (t, *J* = 2.80 Hz, 1H), 6.65 (s, 1H), 6.43 - 6.47 (m, 1H), 3.70 (s, 3H), 3.54 (d, *J* = 11.60 Hz, 1H) ,3.10-3.30 (m, 2H), 2.78-2.81 (m, 1H), 2.50-2.55 (m, 1H), 2.41 (s, 3H), 1.54 - 2.01 (m, 8H), 1.50 (m, 1H), 1.34 (m, 1H), 1.02-1.11 (m, 1H), 0.81 - 0.96 (m, 1H); LC-MS: *m*/*z* = 433.2 [M+H]⁺.

### Example 2: Preparation of compounds 2A, 2B, 2C, and 2D

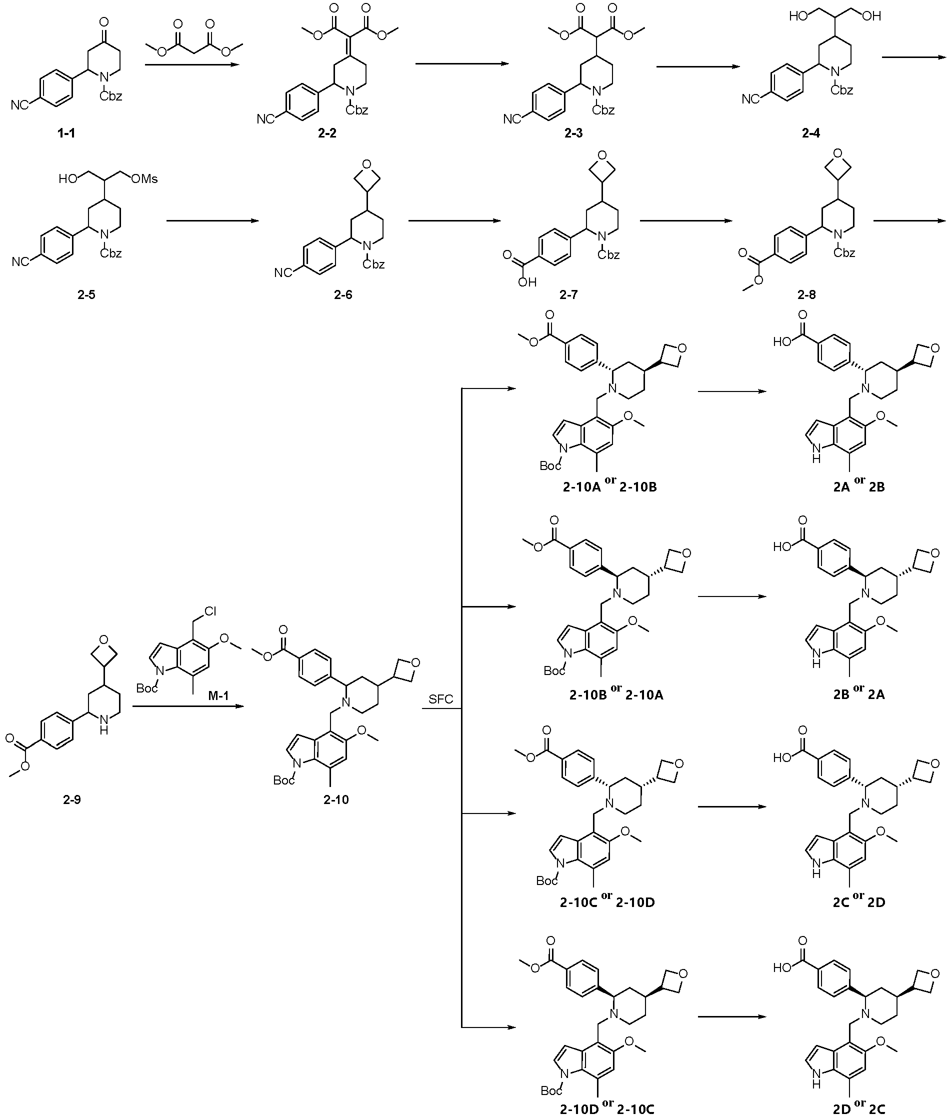

### Step 1

A solution of titanium tetrachloride in dichloromethane (1 M, 1.2 mL) was dissolved in tetrahydrofuran (1 mL) at 0°C, and added with a solution of compound **1-1** (0.20 g) and dimethyl maleate (79.0 mg) in tetrahydrofuran (1 mL). The reaction mixture was stirred at 0°C for 1 hour, added with pyridine (236.6 mg), and stirred continuously at 20°C for 15 hours. The reaction mixture was poured into 10% citric acid to adjust the pH < 7, and extracted with ethyl acetate (20 mL × 3). The organic phases were combined, dried over anhydrous sodium sulfate, filtered, and the filtrate was concentrated under reduced pressure. The residue was purified by silica gel column chromatography (ethyl acetate: petroleum ether = 1:3) to obtain compound **2-2.** ¹H NMR (400 MHz, CDCl₃) δ ppm 7.52 (d, J = 8.40 Hz, 2H), 7.08-7.32 (m, 7H), 5.23-5.42 (m, 1H), 5.06-5.12 (m, 1H), 4.94-5.05 (m, 1H), 4.01-4.11 (m, 1H), 3.66-3.72 (m, 6H), 3.29-3.39 (m, 1H), 3.16-3.27 (m, 1H), 2.74-2.90 (m, 2H), 2.54-2.65 (m, 1H); LC-MS: *m*/*z* = 449.1 [M+H]⁺.

### Step 2

To a solution of compound **2-2** (0.20 g) in methanol (2 mL) was added sodium borohydride (16.9 mg) at 0°C. The reaction mixture was warmed to 25°C, and stirred for 3 hours. The reaction mixture was added with ice water (30 mL) to quench, and extracted with dichloromethane (30 mL × 3). The organic phases were combined, dried over anhydrous sodium sulfate, filtered, and the filtrate was concentrated under reduced pressure to obtain compound **2-3.** LC-MS: *m*/*z* = 451.4 [M+H]⁺.

### Step 3

To a solution of compound **2-3** (1.10 g) in ethanol (15 mL) was added sodium borohydride (923.7 mg) at 25°C. The reaction mixture was heated to 70°C, and stirred for 2 hours. The reaction mixture was cooled to room temperature, added with ice water (50 mL) to quench, and extracted with ethyl acetate (50 mL). The organic phases were combined, dried over anhydrous sodium sulfate, and filtered. The filtrate was concentrated under reduced pressure, and the residue was purified by silica gel column chromatography (ethyl acetate: petroleum ether = 1:0) to obtain compound **2-4.** LC-MS: *m*/*z* = 395.1 [M+H]⁺.

### Step 4

To a solution of compound **2-4** (0.50 g) in tetrahydrofuran (6 mL) was added sodium hydride (purity: 60%, 60.8 mg) at 0°C. The reaction mixture was stirred at 0°C for 0.5 hours, added with methanesulfonyl chloride (0.24 g), warmed to 25°C, and stirred for 1.5 hours. The reaction mixture was added with ice water (5 mL) to quench, and extracted with ethyl acetate (10 mL × 3). The organic phases were combined, and dried over anhydrous sodium sulfate. After filtration, the filtrate was concentrated under reduced pressure, and the residue was purified by silica gel column chromatography (ethyl acetate: petroleum ether = 2:1) to obtain compound **2-5.** LC-MS: *m*/*z* = 473.1 [M+H]⁺.

### Step 5

To a solution of compound **2-5** (1.30 g) in tetrahydrofuran (40 mL) was added sodium hydride (purity: 60%, 330.1 mg) at 0°C, and the reaction mixture was warmed to 20°C and stirred for 1 hour. The reaction mixture was then heated to 60°C, and stirred for 16 hours. The reaction mixture was cooled to room temperature, added with ice water (60 mL) to quench, and extracted with ethyl acetate (10 mL × 3). The organic phases were combined, dried over anhydrous sodium sulfate, and filtered. The filtrate was concentrated under reduced pressure, and the residue was purified by silica gel column chromatography (ethyl acetate: petroleum ether = 2:1) to obtain compound **2-6.** LC-MS: *m*/*z* = 377.1 [M+H]⁺.

### Step 6

To a mixed solution of compound **2-6** (0.55 g) in isopropanol (2 mL), dioxane (2 mL), and water (4 mL) was added barium hydroxide (1.85 g), and the reaction mixture was heated to 100°C and stirred for 14 hours. The reaction mixture was cooled to room temperature, added with 50% potassium bisulfate aqueous solution to adjust the pH < 7, and extracted with ethyl acetate (40 mL × 3). The organic phases were combined, washed once with water (40 mL) and once with saturated brine (45 mL) successively, and dried over anhydrous sodium sulfate. The reaction mixture was filtered, and the filtrate was concentrated under reduced pressure to obtain compound **2-7** (0.58 g). LC-MS: *m*/*z* = 396.1 [M+H]⁺.

### Step 7

To a mixed solution of compound **2-7** (0.58 g) in methanol (1.5 mL) and toluene (4.5 mL) was added (trimethylsilyl)diazomethane (2 M, 1.47 mL) at 20°C, and the reaction mixture was stirred at 20°C for 2 hours. The reaction mixture was cooled to 0°C, added with acetic acid to quench, diluted with water (40 mL), and extracted with ethyl acetate (45 mL × 3). The organic phases were combined, washed with saturated sodium bicarbonate aqueous solution (45 mL), water (45 mL), and saturated brine (50 mL) successively, dried over anhydrous sodium sulfate, and filtered. The filtrate was concentrated under reduced pressure, and the residue was purified by silica gel column chromatography (ethyl acetate: petroleum ether = 2:1) to obtain compound **2-8.** LC-MS: *m*/*z* = 410.1 [M+H]⁺.

### Step 8

To a solution of compound **2-8** (0.40 g) in methanol (5 mL) was added wet palladium on carbon (content: 10%, 0.2 g) at 20°C, and the reaction mixture was stirred at 20°C for 1 hour under hydrogen atmosphere (15 psi). The reaction mixture was filtered, and the filtrate was concentrated under reduced pressure to obtain compound **2-9.** LC-MS: *m*/*z* = 276.1 [M+H]⁺.

### Step 9

To a solution of compound **2-9** (0.26 g) in *N,N*-dimethylformamide (6 mL) was added compound **M-1** (0.65 g), cesium carbonate (769.2 mg), and potassium iodide (156.8 mg) at 20°C. The reaction mixture was stirred at 20°C for 16 hours. The reaction mixture was slowly poured into water (50 mL), and extracted with ethyl acetate (20 mL × 3). The organic phases were combined, dried over anhydrous sodium sulfate, filtered, and the filtrate was concentrated under reduced pressure. The residue was initially purified by silica gel column chromatography (ethyl acetate: petroleum ether = 1:2), and then purified by preparative HPLC (chromatographic column: Phenomenex Gemini-NX 80 × 30 mm × 3 µm; mobile phase: [water (10 mM ammonium bicarbonate)-acetonitrile]; acetonitrile%: 57% to 87%) to obtain compound **2-10,** and chirally separated (column: DAICEL CHIRALPAK AD (250 mm × 30 mm, 10 µm; mobile phase: phase A: carbon dioxide, phase B: [0.1% ammonia water-isopropanol]; B%: 40% to 40%) to obtain compound **2-10A,** compound **2-10B,** compound **2-10C,** and compound **2-10D.**

SFC analysis and detection method: chromatographic column: Chiralpak AD-3 150 mm × 4.6 mm I.D., 3 µm, mobile phase: A: carbon dioxide, B: isopropanol (0.05% diethylamine), gradient: mobile phase B: flowing from 5% to 40% for 5 min, holding at 40% for 5 min, holding at 5% for 2.5 min, flow rate: 2.5 mL/min. Retention time of compound **2-10A:** 4.333 min, ee: 100%; retention time of compound **2-10B:** 4.835 min, ee: 99.5%; retention time of compound **2-10C:** 5.299 min, ee: 99.1%; retention time of compound **2-10D:** 5.698 min, ee: 98.1%. LC-MS: *m*/*z* = 549.2 [M+H]⁺.

### Step 10

To a mixed solution of compound **2-10A** (80.0 mg) in methanol (3 mL) and tetrahydrofuran (1.5 mL) was added lithium hydroxide monohydrate (18.4 mg), and the reaction mixture was heated to 50°C and stirred for 14 hours. The reaction mixture was concentrated under reduced pressure, and the residue was purified by preparative HPLC (chromatographic column: Phenomenex Gemini-NX 80 × 30 mm × 3 µm; mobile phase: [water (10 mM ammonium bicarbonate)-acetonitrile]; acetonitrile%: 15% to 45%) to obtain compound **2A.** ¹H NMR (400 MHz,CD₃OD) δ ppm 8.12 (d, *J* = 8.0 Hz, 1H), 8.12 (d, J = 8.0 Hz, 1H), 7.61 (d, J = 8.0 Hz, 2H), 7.31 (d, J = 2.4 Hz, 1H), 6.77 (s, 1H), 6.33 (br s, 1H), 4.93-4.96 (m, 2H), 4.63 (s, 1H), 4.44-4.80 (m, 2H), 4.22-4.32 (m, 1H), 4.03-4.09 (m, 1H), 3.77 (s, 3H), 3.67-3.75 (m, 1H), 3.06-3.30 (m, 2H), 2.51 (s, 3H), 2.42-2.51 (m, 1H), 2.20-2.37 (m, 1H), 1.96-2.09 (m, 1H), 1.72-1.81(m, 1H), 1.54-1.63 (m, 1H); LC-MS: *m*/*z* = 435.2 [M+H]⁺.

To a mixed solution of compound **2-10B** (80.0 mg) in methanol (3 mL) and tetrahydrofuran (1.5 mL) was added lithium hydroxide monohydrate (18.4 mg), and the reaction mixture was stirred at 50°C for 14 hours. The reaction mixture was concentrated under reduced pressure, and the residue was purified by preparative HPLC (chromatographic column: Phenomenex Gemini-NX 80 × 30 mm × 3 µm; mobile phase: [water (10 mM ammonium bicarbonate)-acetonitrile]; acetonitrile%: 15% to 45%) to obtain compound **2B.** It was identified by two-dimensional NMR that the carbon-hydrogen bond by which piperidine is linked to 4-membered epoxide is an equatorial bond, the carbon-hydrogen bond by which piperidine is linked to benzoic acid is an axial bond, and compound **2B** is in the trans configuration. ¹H NMR (400 MHz, CD₃OD) δ ppm 8.13 (d, J = 8.4 Hz, 2H), 7.61 (d, J = 8.4 Hz, 2H), 7.30 (d, J = 3.2 Hz, 1H), 6.72 (s, 1H), 6.31(d,J = 2.8 Hz, 1H), 4.85-4.91 (m, 2H), 4.36-4.46 (m, 3H), 4.27 (d, *J* = 12.8 Hz, 1H), 4.03 (d, J = 12.8 Hz, 1H), 3.74 (s, 3H), 3.66-3.72 (m, 1H), 3.16-3.25(m, 2H), 2.50 (s, 3H), 2.42-2.47 (m, 1H), 2.27-2.30 (m, 1H), 2.03-2.10 (m, 1H), 1.78-1.81 (m, 1H), 1.59-1.63(m, 1 H); LC-MS: *m*/*z* = 435.2 [M+H]⁺.

To a mixed solution of compound **2-10C** (0.02 g) in methanol (1 mL) and tetrahydrofuran (0.5 mL) was added lithium hydroxide monohydrate (4.6 mg), and the reaction mixture was stirred at 50°C for 14 hours. The reaction mixture was concentrated under reduced pressure, and the residue was purified by preparative HPLC (chromatographic column: Phenomenex Gemini-NX 80 × 30 mm × 3 µm; mobile phase: [water (10 mM ammonium bicarbonate)-acetonitrile]; acetonitrile%: 15% to 45%) to obtain compound **2C**. It was identified by two-dimensional NMR that both the carbon-hydrogen bond by which piperidine is linked to 4-membered epoxide and the carbon-hydrogen bond by which piperidine is linked to benzoic acid are axial bonds, and compound **2C** is in the cis configuration. ¹H NMR (400 MHz, CD₃OD) δ ppm 8.13 (d, J = 8.0 Hz, 2H), 7.61 (d, J = 8.0 Hz, 2H), 7.31 (d, J = 3.2 Hz, 1H), 6.75 (s, 1H), 6.32 (s, 1H), 4.71-4.81 (m, 2H), 4.47-4.56 (m, 2H), 4.29-4.39 (m, 2H), 3.93-3.958 (m, 1H), 3.42-3.48 (m, 1H), 3.75 (s, 3H), 3.11-3.21 (m, 1H), 2.79-2.83 (m, 1H), 2.51 (s, 3H), 2.16-2.29 (m, 1H), 1.91-2.01(m, 1H), 1.79-1.85 (m, 1H), 1.59-1.74 (m, 1H), 1.39-1.46 (m, 1H); LC-MS: *m*/*z* = 435.2 [M+H]⁺.

To a mixed solution of compound **2-10D** (0.02 g) in methanol (1 mL) and tetrahydrofuran (0.5 mL) was added lithium hydroxide monohydrate (4.6 mg), and the reaction mixture was stirred at 50°C for 14 hours. The reaction mixture was concentrated under reduced pressure, and the residue was purified by preparative HPLC (chromatographic column: Phenomenex Gemini-NX 80 × 30 mm × 3 µm; mobile phase: [water (10 mM ammonium bicarbonate)-acetonitrile]; acetonitrile%: 15% to 45%) to obtain compound **2D.** It was identified by two-dimensional NMR that both the carbon-hydrogen bond by which piperidine is linked to 4-membered epoxide and the carbon-hydrogen bond by which piperidine is linked to benzoic acid are axial bonds, and compound **2D** is in the cis configuration. ¹H NMR (400 MHz, CD₃OD) δ ppm 8.14 (d, J = 8.0 Hz, 2H), 7.62 (d, *J* = 8.0 Hz, 2H), 7.31 (d, *J* = 3.2 Hz, 1H), 6.75(s, 1H), 6.32 (s, 1H), 4.71-4.81 (m, 2H), 4.46-4.57(m, 2H), 4.26-4.45(m, 2H), 3.98-4.06(m, 1H), 3.76 (s, 3H), 3.41-3.49 (m, 1H), 3.14-3.26 (m, 1H), 2.76-2.88 (m, 1 H), 2.51(s, 3 H), 2.16-2.29 (m, 1H), 1.95-2.05 (m, 1H), 1.78-1.87 (m, 1H), 1.56-1.75 (m, 1H), 1.36-1.51 (m, 1H); LC-MS: *m*/*z* = 435.2 [M+H]⁺.

### Example 3: Preparation of compounds 3A, 3B, 3C, and 3D

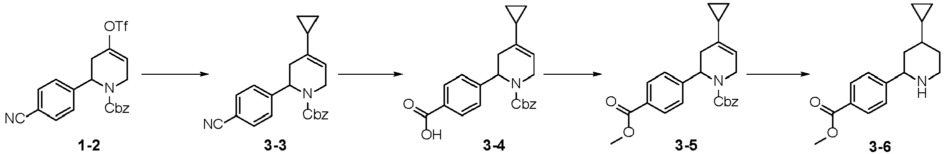

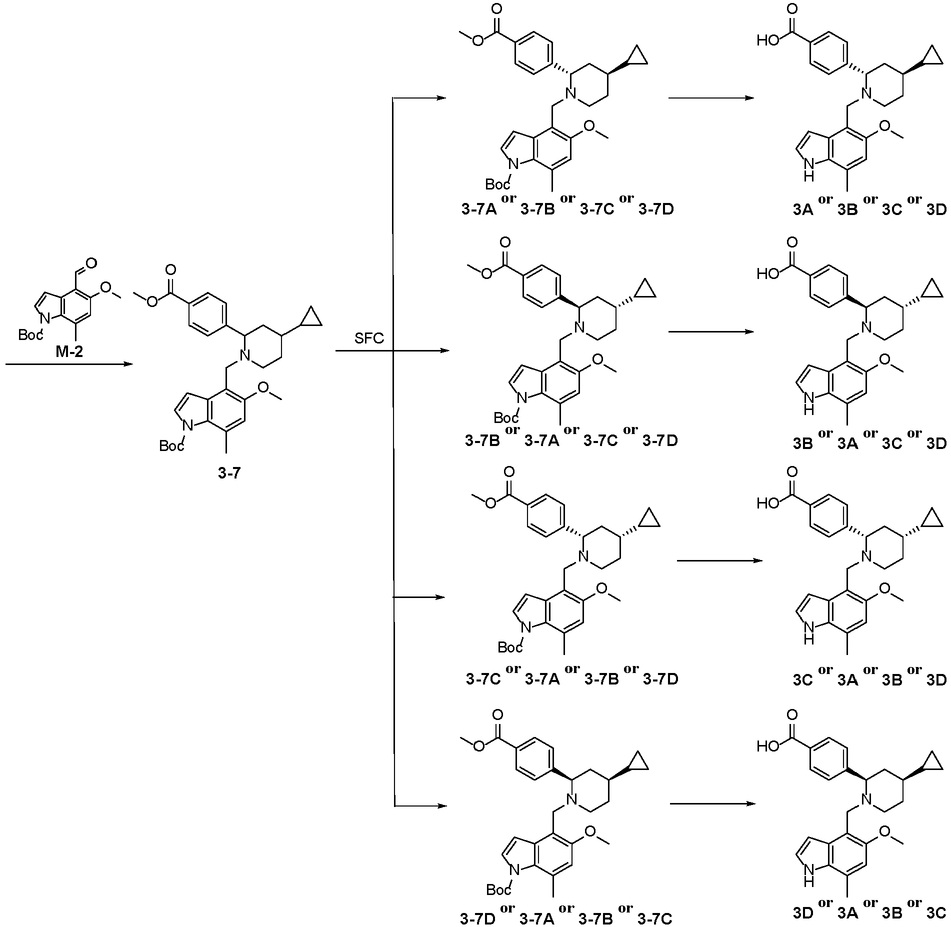

### Step 1

Compound **1-2** (66.0 mg) and cyclopropylboronic acid (24.3 mg) were dissolved in 1,4-dioxane (1 mL), added with potassium phosphate (96.1 mg) and [1,1'-bis(diphenylphosphino)ferrocene]palladium(II)dichloride dichloromethane complex (5.8 mg), and the reaction mixture was stirred at 90°C for 4.5 hours under nitrogen atmosphere. The reaction mixture was concentrated under reduced pressure, added with water (5 mL) to quench, and extracted with ethyl acetate (10 mL). The organic phase was washed with saturated brine, dried over anhydrous sodium sulfate, filtered, and concentrated. The residue was purified by silica gel thin-layer chromatography (petroleum ether: ethyl acetate = 2:1) to obtain compound **3-3.** ¹H NMR (400 MHz, CDCl₃) δ 7.38-7.61 (m, 2H), 7.08-7.33 (m, 7H), 5.44 (br s, 1H), 4.94-5.18 (m, 2H), 3.97-4.24 (m, 1H), 2.75-2.96 (m, 1H), 2.05-2.23 (m, 1H), 1.77-1.80 (m, 1H), 1.31-1.44 (m, 1H), 1.11-1.28 (m, 1H), 0.53-0.68 (m, 2H), 0.37-0.52 (m, 2H); LC-MS: m/z = 359.2 [M+H]⁺.

### Step 2

Compound **3-3** (0.3 g) was dissolved in a mixed solution of isopropanol, 1,4-dioxane, and water (2 mL:2 mL:5 mL). The reaction mixture was added with barium hydroxide (573.6 mg), then heated to 100°C, and stirred at the same temperature for 12 hours. The reaction mixture was cooled to room temperature, added with 50% potassium bisulfate aqueous solution to adjust the pH = 5 to 6, and extracted with dichloromethane (30 mL × 3). The organic phase was dried over anhydrous sodium sulfate, filtered, and concentrated to obtain compound **3-4.** LC-MS: *m*/*z* = 378.0 [M+H]⁺.

### Step 3

Compound **3-4** (50.0 mg) was dissolved in a mixed solution of methanol (1.5 mL) and toluene (0.5 mL), added with (trimethylsilyl)diazomethane (2 M, 199 µL) at room temperature, and the reaction mixture was stirred at the same temperature for 12 hours. The reaction mixture was added with acetic acid (1 mL) and water (10 mL) to quench, and extracted with ethyl acetate (10 mL × 3). The organic phase was washed with saturated brine (10 mL × 1), dried over anhydrous sodium sulfate, filtered, and concentrated. The residue was purified by silica gel thin-layer chromatography (petroleum ether: ethyl acetate = 3:1) to obtain compound **3-5.** ¹H NMR (400 MHz, CDCl₃) δ 7.95-7.97 (m, 2H), 7.28-7.58 (m, 7H), 5.55 (br s, 1H), 5.03-5.24 (m, 2H), 4.09-4.21(m, 1H), 3.92 (s, 3H), 2.84-3.05 (m, 1H), 2.11-2.19(m, 1H), 1.84-1.88 (m, 1H), 1.63 (br s, 1H), 1.37-1.53 (m, 1H), 0.38-0.73 (m, 4H); LC-MS: m/z =392.2 [M+H]⁺.

### Step 4

Compound **3-5** (250.0 mg) was dissolved in a mixed solution of methanol (3 mL) and ethyl acetate (3 mL), added with barium hydroxide (0.10 g), and the reaction mixture was stirred at 15°C for 2 hours under H₂ atmosphere (15 psi). The reaction mixture was directly filtered and concentrated under reduced pressure, and the residue was purified by silica gel thin-layer chromatography (petroleum ether: ethyl acetate = 1:1) to obtain compound **3-6.** LC-MS: m/z =259.9 [M+H]⁺.

### Step 5

Compound **3-6** (0.20 g) was dissolved in 1,2-dichloroethane (5 mL), added with compound **M-2** (133.9 mg) and sodium triacetylcyanoborohydride (269.7 mg), and the reaction mixture was stirred at 15°C for 20 hours. The reaction mixture was added with water (3 mL) to quench, and extracted with ethyl acetate (10 mL × 3). The organic phase was washed with saturated brine (20 mL), dried over anhydrous sodium sulfate, filtered, and concentrated. The residue was purified by HPLC (column: Boston Green ODS 150 × 30 mm×5 µm; mobile phase: [water (0.075% trifluoroacetic acid)-acetonitrile], B (acetonitrile)%: 43% to 73%, 9 min) to obtain compound **3-7.** Compound **3-7** was purified by SFC chiral column chromatography (column: DAICEL CHIRALPAK AD (250 mm × 30 mm, 10 µm; mobile phase: phase A: carbon dioxide, phase B: [0.1% ammonia water-ethanol]; B%: 35% to 35%) to obtain compound **3-7A,** compound **3-7B,** compound **3-7C,** and compound **3-7D.**

SFC detection method: chromatographic column: Chiralpak AD-3 150 mm × 4.6 mm I.D., 3 µm, mobile phase: A: carbon dioxide, B: isopropanol (0.05% diethylamine), gradient: B%: flowing from 5% to 40% for 5 min, holding at 40% for 5 min, holding at 5% for 2.5 min, flow rate: 2.5 mL/min. Retention time of compound **3-7A:** 3.781 min, ee: 100%; retention time of compound **3-7B:** 4.455 min, ee: 74.9%; retention time of compound **3-7C:** 5.030 min, ee: 100%; retention time of compound **3-7D:** 5.527 min, ee: 97.2%.

### Step 6

Compound **3-7A** (10.0 mg) was dissolved in a mixed solution of tetrahydrofuran (0.2 mL) and methanol (0.4 mL) at 20°C, added with lithium hydroxide aqueous solution (1 M, 0.2 mL), and the reaction mixture was stirred at 20°C for 36 hours. The reaction mixture was added with 50% potassium bisulfate aqueous solution to adjust the pH = 6 to 7, and extracted with ethyl acetate (10 mL). The organic phase was collected, dried over anhydrous sodium sulfate, filtered, and concentrated. The residue was purified by HPLC (column: Boston Green ODS 150 × 30 mm×5 µm; mobile phase: [water (0.075% trifluoroacetic acid)-acetonitrile], acetonitrile%: 20% to 60%) to obtain compound **3A**. ¹H NMR (400 MHz, CD₃OD) δ 8.24 (d, *J*=8.2 Hz, 2H), 7.76 (d, *J*=8.2 Hz, 2H), 7.35 (d, *J*=3.2 Hz, 1H), 6.78 (s, 1H), 6.39 (d, *J*=3.0 Hz, 1H), 4.79-4.90(m, 1H), 4.17-4.48 (m, 2H), 3.77 (s, 3H), 3.42-3.70 (m, 2H), 2.52 (s, 3H), 2.21-2.39 (m, 1H), 2.02-2.19 (m, 2H), 1.91-1.96 (m, 1H), 1.33-1.55 (m, 1H), 1.03-1.12 (m, 1H), 0.59-0.69 (m, 2H), 0.09-0.19 (m, 2H); LC-MS: m/z =419.2 [M+H]⁺.

Compound **3-7B** (20.0 mg) was dissolved in a mixed solution of tetrahydrofuran (0.3 mL) and methanol (0.6 mL) at 20°C, added with lithium hydroxide aqueous solution (1 M, 0.3 mL), and the reaction mixture was stirred at 20°C for 36 hours. The reaction mixture was added with 50% potassium bisulfate aqueous solution to adjust the pH = 6 to 7, and concentrated under reduced pressure. The residue was purified by HPLC (column: Boston Green ODS 150 × 30 mm×5 µm; mobile phase: [water (0.075% trifluoroacetic acid)-acetonitrile], acetonitrile%: 20% to 60%) to obtain compound **3B.** ¹H NMR (400 MHz, CD₃OD) δ 8.24 (d, *J* = 8.2 Hz, 2H), 7.76 (d, *J* = 8.2 Hz, 2H), 7.35 (d, *J* = 3.0 Hz, 1H), 6.78 (s, 1H), 6.39 (d, *J* = 3.2 Hz, 1H), 4.79-4.90(m, 1H), 4.19-4.44 (m, 2H), 3.77 (s, 3H), 3.44-3.71 (m, 2H), 2.52 (s, 3H), 2.27-2.39 (m, 1H), 2.03-2.17 (m, 2H), 1.88-1.93 (m, 1H), 1.42-1.53 (m, 1H), 1.07-1.12 (m, 1H), 0.62-0.73 (m, 2H), 0.17-0.26 (m, 2H); LC-MS: m/z =419.2 [M+H]⁺.

Compound **3-7C** (18.0 mg) was dissolved in a mixed solution of tetrahydrofuran (0.3 mL) and methanol (0.6 mL) at 20°C, added with lithium hydroxide monohydrate (1 M, 0.3 mL), and the reaction mixture was stirred at 20°C for 36 hours. The reaction mixture was added with 50% potassium bisulfate aqueous solution to adjust the pH = 6 to 7, and concentrated under reduced pressure. The residue was purified by HPLC (column: Boston Green ODS 150 × 30 mm×5 µm; mobile phase: [water (0.075% trifluoroacetic acid)-acetonitrile], acetonitrile%: 20% to 60%) to obtain compound **3C**. ¹H NMR (400 MHz, CD₃OD) δ 8.23 (d, *J* = 8.0 Hz, 2H), 7.67-7.80 (m, 2H), 7.33 (d, *J* = 3.0 Hz, 1H), 6.77 (s, 1H), 6.34 (d, *J* = 3.0 Hz, 1H), 4.45-4.55(m, 1H), 4.34 (d, *J* = 12.4 Hz, 1H), 4.13 (d, *J* = 12.4 Hz, 1H), 3.76 (s, 3H), 3.53-3.66 (m, 1H), 3.21-3.34 (m, 1H), 2.51 (s, 3H), 2.28-2.39 (m, 1H), 1.88-2.04 (m, 2H), 1.63-1.77 (m, 1H), 1.04-1.21 (m, 1H), 0.55-0.67 (m, 1H), 0.36-0.50 (m, 2H), 0.07-0.28 (m, 2H); LC-MS: m/z =419.2 [M+H]⁺.

Compound **3-7D** (20.0 mg) was dissolved in a mixed solution of tetrahydrofuran (0.3 mL) and methanol (0.6 mL) at 20°C, added with lithium hydroxide monohydrate (1 M, 0.3 mL), and the reaction mixture was stirred at 20°C for 36 hours. The reaction mixture was added with 50% potassium bisulfate aqueous solution to adjust the pH = 6 to 7, and concentrated under reduced pressure. The residue was purified by HPLC (column: Boston Green ODS 150 × 30 mm×5 µm; mobile phase: [water (0.075% trifluoroacetic acid)-acetonitrile], acetonitrile%: 20% to 60%) to obtain compound **3D.** ¹H NMR (400 MHz, CD₃OD) δ 8.24 (br s, 2H), 7.75 (br s, 2H), 7.33 (br s, 1H), 6.76 (br s, 1H), 6.34 (br s, 1H), 4.24-4.58 (m, 2H), 4.09-4.21 (m, 1H), 3.76 (br s, 3H), 3.50-3.66 (m, 1H), 2.59-2.65 (m, 1H), 2.50 (br s, 3H), 2.09-2.30 (m, 1H), 1.86-2.07 (m, 2H), 1.59-1.79 (m, 1H), 1.02-1.30 (m, 1H), 0.35-0.77 (m, 3H), 0.12-0.51 (m, 2H); LC-MS: m/z =419.2 [M+H]⁺.

### Example 4: Preparation of compounds 4A and 4B

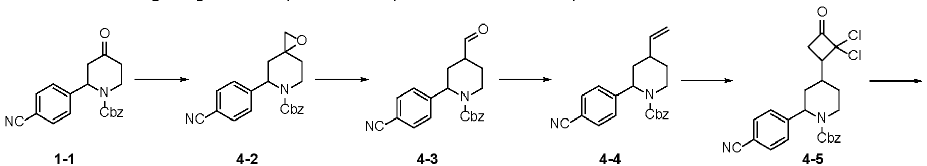

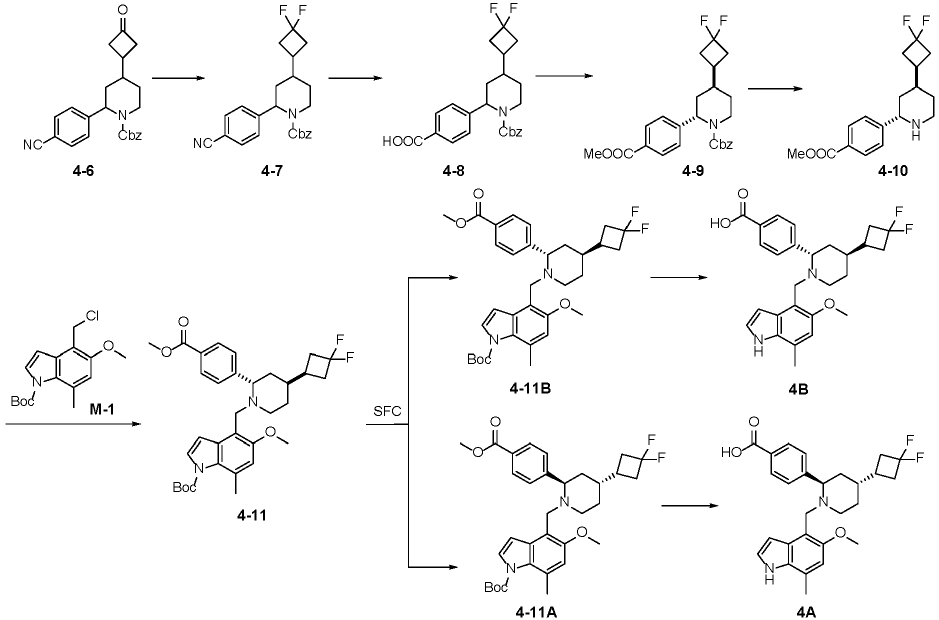

### Step 1

To a solution of trimethylsulfoxonium iodide (2.50 g) in 1,2-dichloroethane (15.0 mL) was added potassium tert-butoxide (1.40 g) at 25°C under nitrogen atmosphere. The reaction mixture was stirred at the same temperature for 0.5 hours, cooled to 0°C, added with a solution of compound **1-1** (3.50 g) in 1,2-dichloroethane (15.0 mL), warmed to 25°C, and stirred for 12 hours. The reaction mixture was added with water (10.0 mL) to quench, and extracted with ethyl acetate (50.0 mL × 3). The organic phase was washed with saturated brine, dried over anhydrous sodium sulfate, filtered, and concentrated under reduced pressure. The residue was purified by silica gel column chromatography (petroleum ether/ethyl acetate, the ratio of petroleum ether: 100% to 35%) to obtain compound **4-2.** LC-MS: m/z =349.1 [M+H] ⁺.

### Step 2

Compound **4-2** (1.70 g) was dissolved in dichloroethane (20.0 mL), then added with boron trifluoride diethyl etherate (1.04 g), and the reaction mixture was stirred at 25°C for 12 hours. The reaction mixture was added with water (5.0 mL) to quench, and extracted with dichloromethane (20.0 mL × 3). The organic phase was washed with saturated brine (20.0 mL × 1), dried over anhydrous sodium sulfate, filtered, and concentrated. The residue was purified by silica gel column chromatography (petroleum ether: ethyl acetate = 1:0 to 2:1) to obtain compound **4-3.** LC-MS: m/z =349.1 [M+H] ⁺.

### Step 3

To a solution of methyltriphenylphosphonium iodide (3.13 g) in tetrahydrofuran (15.0 mL) was added sodium tert-butoxide (744.8 mg) at 25°C under nitrogen atmosphere. The reaction mixture was stirred at 25°C for 1 hour, then added with a solution of compound **4-3** (1.80 g) in tetrahydrofuran (10.0 mL), and stirred continuously for 12 hours. The reaction mixture was added with water (10.0 mL) to quench, and extracted with ethyl acetate (20 mL × 3). The organic phase was dried over anhydrous sodium sulfate, filtered, and concentrated. The residue was purified by silica gel column chromatography (petroleum ether: ethyl acetate = 1:0 to 3:1) to obtain compound **4-4.** ¹H NMR (400 MHz, CDCl₃) δ 7.57-7.71 (m, 2H), 7.29-7.47 (m, 7H), 5.73-5.79 (m, 1H), 5.63-5.70 (m, 1H), 5.20 (br s, 2H), 4.96-5.06 (m, 2H), 4.14-4.35 (m, 1H), 2.76-2.90 (m, 1H), 2.36-2.39 (m, 1H), 2.04-2.18 (m, 1H), 1.79-1.83 (m, 1H), 1.64-1.72 (m, 1H), 1.25-1.46 (m, 1H); LC-MS: m/z =347.1 [M+H] ⁺.

### Step 4

Compound **4-4** (1.20 g), zinc-copper couple (4.47 g), and phosphorus oxychloride (584.3 mg) were dissolved in dimethoxyethane (50.0 mL) at 20°C under nitrogen atmosphere, then added with trichloroacetyl chloride (3.15 g), and the reaction mixture was stirred at 20°C for 12 hours. The reaction mixture was concentrated under reduced pressure, and the residue was purified by flash silica gel column chromatography (ethyl acetate/petroleum ether, the ratio of ethyl acetate: 0 to 50%) to obtain compound **4-5.** LC-MS: m/z =457, 459 [M+H]⁺.

### Step 5

Compound **4-5** (1.50 g) was dissolved in dioxane (2.0 mL) at 20°C, and added with glacial acetic acid (15.75 g) and zinc powder (2.14 g). After the addition was completed, the reaction mixture was heated to 80°C, and stirred at the same temperature for 1 hour. The reaction mixture was filtered, and the filtrate was concentrated under reduced pressure. The residue was purified by flash silica gel column chromatography (ethyl acetate/petroleum ether, the ratio of ethyl acetate: 0 to 50%) to obtain compound **4-6.** ¹H NMR (400 MHz, CDCl₃) δ :7.63 (d, J=8.4 Hz, 2H), 7.21-7.48 (m, 7H), 5.64 (br s, 1H), 5.19 (br s, 2H), 4.28 (br s, 1H), 3.03-3.20 (m, 2H), 2.64-2.87 (m, 3H), 2.30-2.43 (m, 1H), 2.02-2.19 (m, 1H), 1.67 (m, 2H), 1.21-1.41 (m, 2H).

### Step 6

Compound **4-6** (1.2 g) was dissolved in dichloroethane (30.0 mL) at 15°C. The reaction mixture was cooled to -15°C, added with diethylaminosulfur trifluoride (2.49 g), slowly warmed to 15°C, and stirred continuously for 12 hours. The reaction mixture was slowly poured into ice water (100.0 mL) to quench, added with saturated sodium bicarbonate aqueous solution to adjust the pH to 7, and extracted with dichloromethane (100 mL × 3). The organic phases were combined and concentrated under reduced pressure. The residue was purified by flash silica gel column chromatography (ethyl acetate/petroleum ether, the ratio of ethyl acetate: 0 to 50%) to obtain compound **4-7.** ¹H NMR (400 MHz, CDCl₃) δ 7.64 (d, J=8.4 Hz, 2H), 7.10-7.50 (m, 7H), 5.62 (br s, 1H), 5.19 (br s, 2H), 4.24 (br s, 1H), 2.74-2.77 (m, 1H), 2.52-2.72 (m, 2H), 2.20-2.35 (m, 1H), 2.06-2.22 (m, 2H), 1.78-1.91 (m, 1H), 1.59-1.68 (m, 1H), 1.50-1.57 (m, 1H), 1.23-1.39 (m, 1H), 1.07-1.21 (m, 1H).

### Step 7

Compound **4-7** (1.00 g) was dissolved in a mixed solution of isopropanol (5.0 mL), dioxane (5.0 mL), and water (10.0 mL) at 20°C. The reaction mixture was added with barium hydroxide (960.1 mg), heated to 100°C, and stirred at the same temperature for 12 hours. The reaction mixture was cooled to room temperature, poured into water (100.0 mL) to quench, added with 1 M hydrochloric acid to adjust the pH to 5 to 6, and extracted with ethyl acetate (100 mL × 3). The organic phases were combined and concentrated under reduced pressure to obtain compound **4-8.** LC-MS: m/z =430 [M+H]⁺.

### Step 8

Compound **4-8** (0.98 g) was dissolved in a mixed solution of methanol (5.0 mL) and toluene (15.0 mL) at 20°C. The reaction mixture was then slowly added with (trimethylsilyl)diazomethane (2 M, 2.45 mL), and stirred at 20°C for 0.5 hours. The reaction mixture was slowly added dropwise with acetic acid (2.0 mL) to quench and then concentrated under reduced pressure. The concentrate was purified by flash silica gel column chromatography (ethyl acetate/petroleum ether, the ratio of ethyl acetate: 0 to 50%) to obtain compound **4-9.** Two-dimensional NMR spectrum (HSQC, COZY, NOE) analysis results show that the C-H by which piperidine is linked to difluorocyclobutane is an axial bond, and has a NOE correlation with the two hydrogens at the α-position of the benzene ring, so the relative configuration of difluorocyclobutane and methyl benzoate is determined to be trans configuration. ¹H NMR (400 MHz, CDCl₃) δ 8.04 (br d, J=8.4 Hz, 2H), 7.10-7.50 (m, 7H), 5.64 (br s, 1H), 5.21 (s, 2H), 4.27 (br s, 1H), 3.94 (s, 3H), 2.77-2.92 (m, 1H), 2.51-2.76 (m, 2H), 2.27-2.41 (m, 1H), 2.08-2.26 (m, 2H), 1.73-1.92 (m, 1H), 1.47-1.69 (m, 2H), 1.30-1.45 (m, 1H), 1.08-1.23 (m, 1H); LC-MS: m/z =444 [M+H] ⁺.

### Step 9

Compound **4-9** (900.0 mg) was dissolved in ethyl acetate (15.0 mL) at 25°C, and then added with a solution of palladium hydroxide (180.0 mg) in methanol (5.0 mL). The system was replaced with hydrogen three times to maintain a hydrogen pressure of 15 psi, and the reaction mixture was stirred at the same temperature for 2 hours. After filtration, the filtrate was concentrated under reduced pressure to obtain compound **4-10.** ¹H NMR (400 MHz, CDCl₃) δ 8.00 (d, J=8.4 Hz, 2H), 7.45 (d, J=8.4 Hz, 2H), 3.92-3.98 (m, 1H), 3.91 (s, 3H), 2.953-2.96 (m, 2H), 2.64-2.76 (m, 2H), 2.38-2.52 (m, 1H), 2.05-2.25 (m, 2H), 1.80-1.90 (m, 1H), 1.70-1.79 (m, 2H), 1.61-1.68 (m, 1H), 1.43-1.52 (m, 1H); LC-MS: m/z =310 [M+H] ⁺.

### Step 10

Compound **4-10** (70.0 mg) and compound **M-1** (180.0 mg) were dissolved in N,N-dimethylformamide (3.0 mL) at 25°C, then added with cesium carbonate (221.2 mg) and potassium iodide (37.6 mg, 226.28 µmol, 1 eq.), and the reaction mixture was stirred at the same temperature for 12 hours. The reaction mixture was poured into water (100 mL) to quench, and extracted with ethyl acetate (70 mL × 3). The organic phases were combined and concentrated under reduced pressure. The residue was purified by silica gel thin-layer chromatography (petroleum ether: ethyl acetate = 3:1) to obtain compound **4-11**, and then chirally separated (chiral column: Phenomenex-Cellulose-2 (250 mm × 30 mm, 10 µm)); mobile phase: [A: carbon dioxide, B: ethanol (0.1% ammonia water)]; gradient B%: 20% to 20%) to obtain compound **4-11A** and compound **4-11B.**

SFC analysis and detection method: chromatographic column: Cellulose 2 150 mm × 4.6 mm I.D., 5 µm, mobile phase: A: carbon dioxide, B: ethanol (0.05% diethylamine), gradient: B%: flowing from 5% to 40% for 5 min, holding at 40% for 5 min, holding at 5% for 2.5 min, flow rate: 2.5 mL/min. Retention time of compound **4-11A:** 3.267 min, retention time of compound **4-11B:** 3.514 min. LC-MS: m/z =583 [M+H] ⁺.

### Step 11

### Compound 4A:

Compound **4-11A** (50.0 mg) was dissolved in a mixed solution of tetrahydrofuran (1.0 mL), methanol (2.0 mL), and water (1.0 mL) at 20°C. The reaction mixture was then added with lithium hydroxide (40.0 mg), heated to 50°C, and stirred for 12 hours. The reaction mixture was poured into water (20.0 mL), added with 4 M acetic acid aqueous solution to adjust the pH to 4 to 5, and extracted with ethyl acetate (30 mL × 3). The organic phase was collected, washed with saturated brine (20.0 mL), dried over anhydrous sodium sulfate, filtered, and concentrated. The residue was dissolved in a mixed solution of water (10.0 mL) and acetonitrile (2.0 mL), and freeze-dried to obtain compound **4A** (SFC detection method: chromatographic column: Chiralpak AD-3 150 mm × 4.6 mm I.D., 3 µm, mobile phase: A: carbon dioxide, B: isopropanol (0.05% diethylamine), gradient: B%: flowing from 5% to 40% for 5.5 min, holding at 40% for 3 min, holding at 5% for 1.5 min, flow rate: 2.5 mL/min, retention time: 6.521 min, ee: 99.7%). ¹H NMR (400 MHz, DMSO-*d₆*) δ: 10.80 (br s, 1H), 7.89 (d, J=8.4 Hz, 2H), 7.52 (br d, J=8.0 Hz, 2H), 7.24 (t, J=2.8 Hz, 1H), 6.64 (s, 1H), 6.48 (t, J=2.4 Hz, 1H), 3.70 (s, 3H), 3.50-3.60 (m, 2H), 2.67-2.77 (m, 2H), 2.41 (s, 3H), 2.08-2.25 (m, 4H), 1.71-1.80 (m, 1H), 1.58-1.68 (m, 1H), 1.48-1.58 (m, 3H), 1.34-1.37 (m, 2H); LC-MS: m/z =469 [M+H] ⁺.

### Compound 4B:

Compound **4-11B** (46.0 mg) was dissolved in a mixed solution of tetrahydrofuran (1.0 mL), methanol (2.0 mL), and water (1.0 mL) at 20°C. The reaction mixture was then added with lithium hydroxide (40.0 mg), heated to 50°C, and stirred for 12 hours. The reaction mixture was poured into water (20.0 mL), added with 4 M acetic acid aqueous solution to adjust the pH to 4 to 5, and extracted with ethyl acetate (30 mL × 3). The organic phase was collected, washed with saturated brine (20.0 mL), dried over anhydrous sodium sulfate, filtered, and concentrated. The residue was dissolved in a mixed solution of water (10.0 mL) and acetonitrile (2.0 mL), and freeze-dried to obtain compound **4B** (SFC detection method: chromatographic column: Chiralpak AD-3 150 mm × 4.6 mm I.D., 3 µm, mobile phase: A: carbon dioxide, B: isopropanol (0.05% diethylamine), gradient: B%: flowing from 5% to 40% for 5.5 min, holding at 40% for 3 min, holding at 5% for 1.5 min, flow rate: 2.5 mL/min, retention time: 4.832 min, ee: 96.0%). ¹H NMR (400 MHz, DMSO-*d₆*) δ:10.80 (br s, 1H), 7.93 (d, J=8.0 Hz, 2H), 7.61 (br d, J=7.4 Hz, 2H), 7.24 (t, J=2.8 Hz, 1H), 6.64 (s, 1H), 6.48 (t, J=2.0 Hz, 1H), 3.70 (s, 3H), 3.45-3.65 (m, 2H), 2.67-2.78 (m, 2H), 2.41 (s, 3H), 2.11-2.28 (m, 4H), 1.71-1.80 (m, 1H), 1.62 (br s, 1H), 1.45-1.58 (m, 3H), 1.30-1.40 (m, 2H); LC-MS: m/z =469 [M+H]⁺.

### Example 5: Preparation of compound 4C

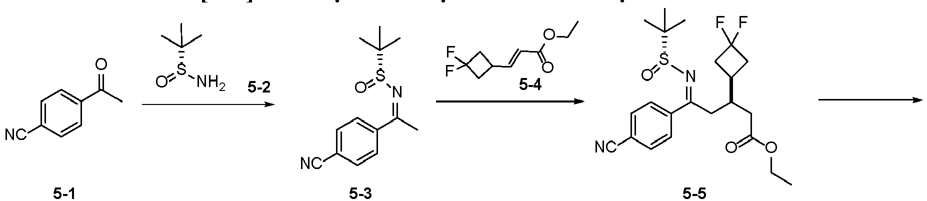

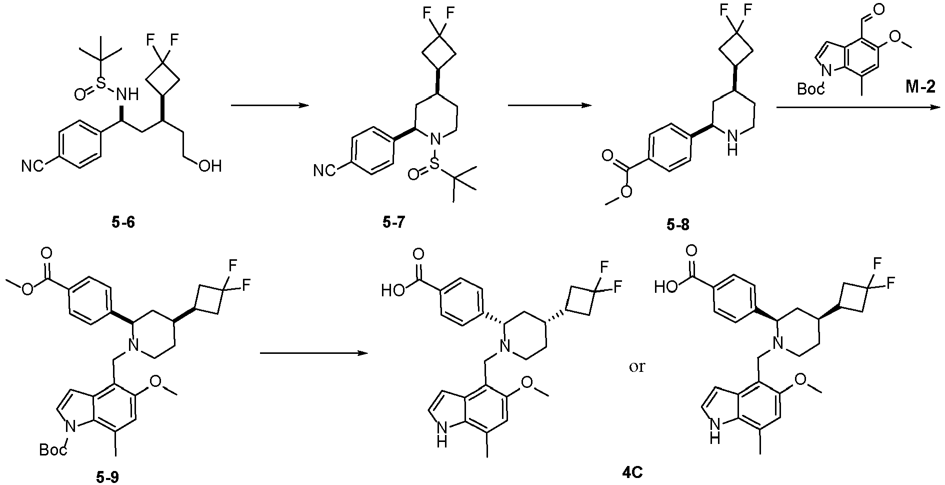

### Step 1

Compound **5-1** (1.00 kg) and compound **5-2** (919.93 g) were dissolved in 2-methyltetrahydrofuran (10.0 L) at 25°C, and titanium ethoxide (4.81 kg) was added in batches. The reaction mixture was heated to 80°C under nitrogen atmosphere and stirred for 16 hours. The reaction mixture was poured into ice water (15.0 L), filtered, and the filter cake was washed with 2-methyltetrahydrofuran (2.0 L × 3). The filtrate was collected, washed with saturated brine (3.0 L), dried over anhydrous sodium sulfate, filtered, and concentrated under reduced pressure to remove 2-methyltetrahydrofuran (8.0 L). The remaining solution was poured into petroleum ether (10.0 L) at 0°C, stirred for 1 hour, and filtered. The filter cake was washed with petroleum ether (1.0 L × 3), and then dried under reduced pressure to obtain compound **5-3.** ¹H NMR (400 MHz, CDCl₃) δ:7.96-7.98 (m, 2H), 7.73-7.75 (m, 2H), 2.81 (s, 3H), 1.34 (s, 9H).

### Step 2

To a solution of compound **5-3** (5.00 g) in 2-methyltetrahydrofuran (100.0 mL) was added lithium diisopropylamide (2 M tetrahydrofuran solution, 12.08 mL) dropwise at -20°C under nitrogen atmosphere. After the dropwise addition was completed, the reaction mixture was stirred at -20°C to -10°C for 0.5 hours, added with a solution of compound **5-4** (5.74 g) in 2-methyltetrahydrofuran (25.0 mL), slowly warmed, and stirred at -20°C to -10°C for 7 hours. The reaction mixture was added with saturated ammonium chloride aqueous solution (30.00 mL) to quench, diluted with water (200.0 mL), and extracted with ethyl acetate (100.0 mL × 2). The organic phases were combined, washed with saturated brine (100.0 mL), dried over anhydrous sodium sulfate, and filtered. The filtrate was concentrated under reduced pressure, and the residue was purified by silica gel column chromatography (petroleum ether: ethyl acetate, the ratio of ethyl acetate: 0 to 20%) to obtain compound **5-5.** LC-MS: m/z =439.1

### Step 3

Compound **5-5** (5.00 g) was dissolved in tetrahydrofuran (50.0 mL) at 0°C, then added with sodium borohydride (0.62 g), and the reaction mixture was stirred at 0°C for 3 hours. The reaction mixture was added with water (30.0 mL) to quench, and extracted with ethyl acetate (30 mL × 2). The organic phase was washed with saturated brine (30 mL × 1), dried over anhydrous sodium sulfate, and filtered. The filtrate was concentrated under reduced pressure, and the residue was purified by silica gel column chromatography (petroleum ether: ethyl acetate, the ratio of ethyl acetate: 0 to 100%) to obtain compound **5-6.** LC-MS: m/z =399.2 [M+H]⁺.

### Step 4

To a solution of compound **5-6** (600.0 mg) in tetrahydrofuran (15.0 mL) was added triphenylphosphine (1.18 g) and diisopropyl azodicarboxylate (913.3 mg) at 25°C under nitrogen atmosphere. The reaction mixture was stirred at 25°C for 18 hours. The reaction mixture was concentrated under reduced pressure, and the residue was purified by silica gel column chromatography (petroleum ether: ethyl acetate = 0 to 50%) to obtain compound **5-7.** LC-MS: m/z =381.1 [M+H]⁺.

### Step 5

Compound **5-7** (200.0 mg) was dissolved in methanol (1.0 mL) at 20°C under nitrogen atmosphere, and the reaction mixture was added with concentrated H₂SO₄ (1.0 mL). The reaction mixture was heated to 80°C and stirred for 3 hours. The reaction mixture was cooled to room temperature, diluted with water (50.0 mL), added with saturated sodium bicarbonate aqueous solution to adjust the pH to 7 to 8, and extracted with ethyl acetate (50.0 mL × 2). The organic phases were combined, washed with saturated brine (50.0 mL), dried over anhydrous sodium sulfate, and filtered. The filtrate was concentrated under reduced pressure, and the residue was purified by a silica gel thin-layer chromatography plate (dichloromethane: methanol, the ratio of methanol: 0 to 10%) to obtain compound **5-8.** LC-MS: m/z =310.2 [M+H]⁺.

### Step 6

To a solution of compound **5-8** (50.0 mg) and compound **M-2** (51.4 mg) in 1,2-dichloroethane (1.0 mL) was added sodium triacetoxyborohydride (137.0 mg) at 25°C. The reaction mixture was stirred at 25°C for 16 hours. The reaction mixture was diluted with water (200 mL), and extracted with ethyl acetate (50 mL × 2). The organic phases were combined, washed with water (100 mL × 2), dried over anhydrous sodium sulfate, and filtered. The filtrate was concentrated under reduced pressure, and purified by silica gel column chromatography (petroleum ether: ethyl acetate, the ratio of ethyl acetate: 0 to 15%) to obtain compound **5-9.** LC-MS: m/z =583.4 [M+H]⁺.

### Step 7

Compound **5-9** (30.0 mg) was dissolved in tetrahydrofuran (0.5 mL) and methanol (0.5 mL) at 25°C, added with lithium hydroxide monohydrate (13.0 mg), and the reaction mixture was stirred at 50°C for 16 hours. The reaction mixture was cooled to room temperature, added with acetic acid (0.1 mL) to quench, diluted with water (50.0 mL), and extracted with ethyl acetate (25.0 mL × 2). The organic phases were combined, dried over anhydrous sodium sulfate, filtered, and dried under reduced pressure to obtain compound **4C.**

LC-MS: m/z =469.1 [M+H]⁺.

### Example 6: Preparation of compound 4D

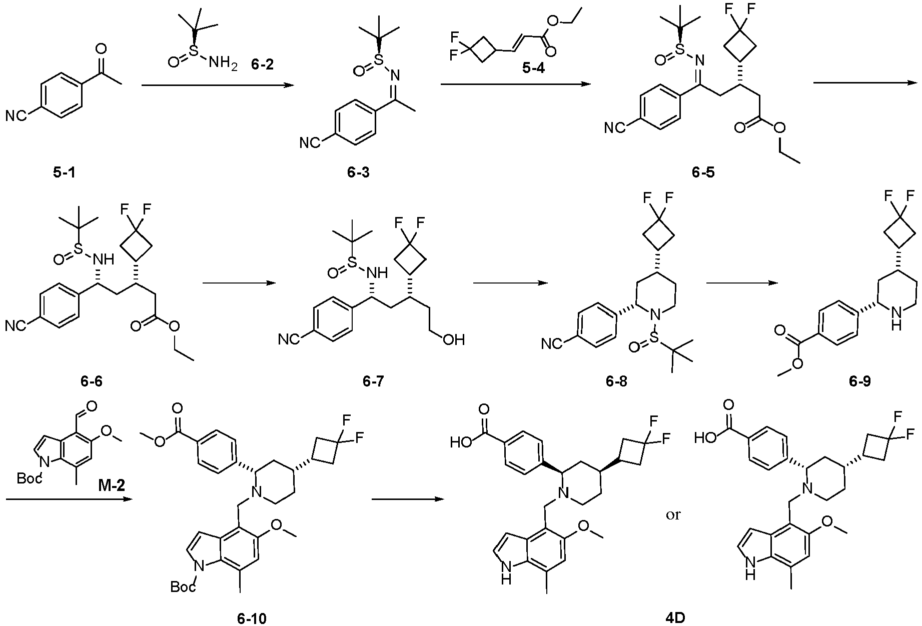

### Step 1

Compound **5-1** (50.00 g) and compound **6-2** (45.92 g) were dissolved in 2-methyltetrahydrofuran (500.0 mL) at 25°C, and titanium ethoxide (235.72 g) was added in batches. The reaction mixture was heated to 80°C under nitrogen atmosphere and stirred for 16 hours. The reaction mixture was poured into ice water (15.0 L), filtered, and the filter cake was washed with 2-methyltetrahydrofuran (3.0 L). The filtrate was collected, dried over anhydrous sodium sulfate, filtered, and concentrated under reduced pressure to remove 2-methyltetrahydrofuran (400.0 mL). The remaining solution was poured into petroleum ether (200.0 mL) at 0°C, stirred for 1 hour, and filtered. The filter cake was collected, and dried under reduced pressure to obtain compound **6-3.** ¹H NMR (400 MHz, CDCl₃) δ:7.95-7.97 (d, J=8.0 Hz, 2H), 7.72-7.74 (d, J=8.0 Hz, 2H), 2.79(s, 3H), 1.33(s, 9H).

### Step 2

To a solution of compound **6-3** (25.00 g) in 2-methyltetrahydrofuran (500.0 mL) was added lithium diisopropylamide (2 M tetrahydrofuran solution, 60.40 mL) dropwise at -20°C under nitrogen atmosphere. After the dropwise addition was completed, the reaction mixture was stirred at -20°C to -15°C for 1 hour, added with a solution of compound **5-4** (35.35 g) in 2-methyltetrahydrofuran (125.0 mL), slowly warmed, and stirred at -20°C to -15°C for 4 hours. The reaction mixture was added with saturated ammonium chloride aqueous solution (60.00 mL) to quench, diluted with water (300.0 mL), and extracted with ethyl acetate (200.0 mL × 2). The organic phases were combined, washed with water (200.0 mL × 2), dried over anhydrous sodium sulfate, and filtered. The filtrate was concentrated under reduced pressure, and the residue was purified by silica gel column chromatography (petroleum ether: ethyl acetate, the ratio of ethyl acetate: 0 to 13%) to obtain compound **6-5.**

### Step 3

Compound **6-5** (5.20 g) was dissolved in tetrahydrofuran (52.0 mL) at 0°C, then added with sodium borohydride (0.50 g), and the reaction mixture was stirred at 0°C for 1 hour. The reaction mixture was added with saturated ammonium chloride solution (50.0 mL) to quench, diluted with water (200.0 mL), extracted with ethyl acetate (100.0 mL × 2), dried over anhydrous sodium sulfate, and filtered. The filtrate was concentrated under reduced pressure, and the residue was purified by silica gel column chromatography (petroleum ether: ethyl acetate, the ratio of ethyl acetate: 0 to 33%) to obtain compound **6-6.** LC-MS: m/z =441.1 [M+H] ⁺.

### Step 4

Compound **6-6** (3.30 g) was dissolved in tetrahydrofuran (15.0 mL) at -15°C, then added with lithium triisobutylhydroborate (1 M tetrahydrofuran solution, 5.02 mL), and the reaction mixture was stirred at -15°C for 4 hours. The reaction mixture was added with saturated ammonium chloride solution (50.0 mL) to quench, diluted with water (200.0 mL), and extracted with ethyl acetate (50.0 mL × 3). The organic phases were combined, dried over anhydrous sodium sulfate, and filtered. The filtrate was concentrated under reduced pressure, and the residue was purified by silica gel column chromatography (dichloromethane: methanol, the ratio of methanol: 0 to 5%) to obtain compound **6-7.** LC-MS: m/z =399.2 [M+H]⁺.

### Step 5

To a solution of compound **6-7** (2.30 g) in tetrahydrofuran (46.0 mL) was added triphenylphosphine (5.30 g) and diisopropyl azodicarboxylate (4.08 g) at 25°C. The reaction mixture was stirred at 25°C for 16 hours under nitrogen atmosphere. The reaction mixture was concentrated under reduced pressure, and the residue was purified by silica gel column chromatography (petroleum ether: ethyl acetate, the ratio of ethyl acetate: 0 to 40%) to obtain compound **6-8.** LC-MS: m/z =381.1 [M+H]⁺.

### Step 6

Compound **6-8** (500.0 mg) was dissolved in methanol (5.0 mL) at 20°C under nitrogen atmosphere, and the reaction mixture was added with concentrated H₂SO₄ (5.0 mL). The reaction mixture was heated to 80°C and stirred for 16 hours. The reaction mixture was cooled to room temperature, added with saturated sodium bicarbonate aqueous solution to adjust the pH to 7 to 8, and extracted with ethyl acetate (50.0 mL × 2). The organic phases were combined, dried over anhydrous sodium sulfate, and filtered. The filtrate was concentrated under reduced pressure, and the residue was purified by a silica gel thin-layer chromatography plate (dichloromethane: methanol = 0 to 10%) to obtain compound **6-9.** LC-MS: m/z =310.1 [M+H]⁺.

### Step 7

To a solution of compound **6-9** (130.0 mg) and compound **M-2** (133.7 mg) in 1,2-dichloroethane (3.0 mL) was added sodium triacetoxyborohydride (356.3 mg) at 25°C. The reaction mixture was stirred at 25°C for 16 hours. The reaction mixture was diluted with water (200.0 mL) and extracted with ethyl acetate (50 mL × 2). The organic phases were combined, washed with water (100 mL × 2), dried over anhydrous sodium sulfate, and filtered. The filtrate was concentrated under reduced pressure, and purified by silica gel column chromatography (petroleum ether: ethyl acetate, the ratio of ethyl acetate: 0 to 15%) to obtain compound **6-10.** LC-MS: m/z =583.4 [M+H]⁺.

### Step 8

Compound **6-10** (30.0 mg) was dissolved in tetrahydrofuran (0.5 mL) and methanol (0.5 mL) at 25°C, added with lithium hydroxide monohydrate (13.0 mg), and the reaction mixture was stirred at 50°C for 16 hours. The reaction mixture was cooled to room temperature, added with acetic acid (0.1 mL) to quench, diluted with water (50.0 mL), and extracted with ethyl acetate (25.0 mL × 2). The organic phases were combined, dried over anhydrous sodium sulfate, filtered, and dried under reduced pressure to obtain compound **4D.** LC-MS: m/z =469.1 [M+H]⁺.

### Bioassay data:

### Experimental example 1: Wieslab alternative complement pathway activation inhibition (enzyme activity test)

The objective of this experiment is to determine the inhibitory activity of the compounds of the present disclosure on the alternative complement pathway in human serum by using the Wieslab^{®} complement system alternative pathway kit.

### Experimental scheme:

Serum was diluted with diluent (1:23). Drugs were added to the diluted serum with 8 concentration gradients of up to 10 mM or 50 mM in a 5-fold serial dilution. The mixture was incubated at room temperature for 15 min. The mixture of compound and serum was added to a 96-well plate (100 µL/well) provided in the kit, and activated at 37°C for 1 hour. The plate was washed three times with wash buffer. The plate was added with detection antibody (100 µL) provided in the kit and incubated at room temperature for 30 min. The plate was washed three times with wash buffer. The plate was added with substrate (100 µL) and incubated at room temperature for 30 min. The absorbance was detected at 405 nM on a microplate reader.

Experimental results: The results of the inhibitory activity of the test compounds on the alternative complement pathway are shown in Table 1.

**Table 1: Results of in vitro enzyme activity screening test**

| Compound | **AP MAC IC₅₀ (nM)** |
|---|---|
| Compound **3A** | 23.6 |
| Compound **3C** | 51.7 |
| Compound **4B** | 40.7 |
| **LNP023** | 38.0* |

| | |
|---|---|
| Note: * represents the average of two tests | |

Conclusion: The compounds of the present disclosure have significant inhibitory activity on the activation of the alternative pathway in human serum.

### Experimental example 2: Pharmacokinetic study in mice

Experimental objective: To investigate in vivo plasma pharmacokinetics of the compounds of the present disclosure after a single intravenous injection and intragastric administration in male C57BL/6J mice.

Experimental animals: male C57BL/6J mice, 7 to 9 weeks old, weighing 17 to 23 g; supplier: Shanghai Sipple-Bikai Laboratory Animal Co., Ltd.

Experimental procedure: injection administration (IV): a dose of 1 mg/kg (vehicle: 10% PG/5% Solutol/85% PBS (1× pH 7.4)); oral administration (PO): a dose of 10 mg/kg (vehicle: 30% PEG300/10% Cremphor EL/60% PBS (1× pH 7.4))
Sample collection: 0.03 mL of blood samples were collected by puncture of the saphenous vein in experimental animals at each time point, and the actual blood collection time was recorded. All blood samples were added to 1.5 mL commercial EDTA-K2 anticoagulant tubes (supplied by Jiangsu Kangjian Medical Apparatus Co., Ltd.). After collection, within half an hour, the blood sample was centrifuged at 4°C, 3000 g for 10 min to aspirate the supernatant plasma, which was put in dry ice immediately, and stored in a refrigerator at -80°C for LC-MS/MS analysis.

Data analysis: Plasma concentrations were processed using the non-compartmental model of WinNonlin^{™} Version 6.3 (Pharsight, Mountain View, CA) pharmacokinetic software, and pharmacokinetic parameters CI, T_{1/2}, Cₘₐₓ, and AUC₀₋ₗₐₛₜ were calculated using the linear/log trapezoidal method. The results are shown in Table 2.

**Table 2: Comparison of PK results between the compounds of the present disclosure and the reference compound**

| Compound | IV (1 mpk) | | PO (10 mpk) | |
|---|---|---|---|---|
| | Cl (mL/Kg/min) | T_{1/2} (h) | Cₘₐₓ (nM) | AUC₀₋ₗₐₛₜ (nM·h) |
| Compound **3A** | 12.0 | 3.9 | 4772 | 11156 |
| Compound **3C** | 11.1 | 3.4 | 6320 | 23300 |
| Compound **4B** | 6.3 | 4.9 | 3495 | 14850 |
| **LNP023** | 18.4 | 1.3 | 2495 | 9296 |

| | | | | |
|---|---|---|---|---|
| Note: C!: apparent clearance; T_{1/2}: time required to clear half of the compound; Cₘₐₓ: peak concentration; AUC₀₋ₗₐₛₜ: integrated area of concentration during a period from 0 to last sampling time. | | | | |

Experimental conclusion: The results of the pharmacokinetic study in mice show that the compounds of the present disclosure have a longer half-life than that of the reference compound, a significantly higher oral plasma exposure than that of the reference compound **LNP023**, and the compounds of the present disclosure have good pharmacokinetic properties.

### Experimental example 3: Pharmacokinetic study in rats

Experimental objective: To investigate in vivo plasma pharmacokinetics of the compounds of the present disclosure after a single intravenous injection and intragastric administration in male Wistar Han rats.

Experimental animals: male Wistar Han rats, 7 to 9 weeks old, weighing 231.03 to 243.2 g; supplier: Beijing Vital River Laboratory Animal Technology Co., Ltd.

Experimental procedure: injection administration (IV): a dose of 1 mpk (vehicle: 30% PEG300/10% Cremphor EL/60% PBS (1× pH 7.4)); oral administration (PO): a dose of 5 mpk or 30 mpk (vehicle: 30% PEG300/10% Cremphor EL/60% PBS (1× pH 7.4))
Sample collection: About 0.3 mL of blood samples were collected by puncture of the jugular vein in experimental animals at each time point, and the actual blood collection time was recorded. All blood samples were added to 1.5 mL commercial EDTA-K2 anticoagulant tubes (supplied by Jiangsu Kangjian Medical Apparatus Co., Ltd.). After collection, the blood sample was centrifuged at 4°C, 3200 g for 10 min to aspirate the supernatant plasma, which was put in dry ice immediately, and stored in a refrigerator at -80°C for LC-MS/MS analysis.

Data analysis: Plasma concentrations were processed using the non-compartmental model of Phoenix WinNonlin 6.3 pharmacokinetic software, and pharmacokinetic parameters CI, Cₘₐₓ, T_{1/2}, and AUC₀₋ₗₐₛₜ were calculated using the linear/log trapezoidal method. The results are shown in Table 3.

**Table 3: Comparison of PK results between the compounds of the present disclosure and the reference compound in rats**

| Compound | IV (1 mpk) | | PO (5 mpk or 30 mpk) | | |
|---|---|---|---|---|---|
| | Cl (mL/Kg/min) | T_{1/2} (h) | Cₘₐₓ (nM) | AUC₀₋ₗₐₛₜ (nM·h) | F% |
| Compound **3C** | 4.6 | 3.8 | 838 | 5399^{a} | 62% |
| Compound **4B** | 3.5 | 5.7 | 772 | 6253^{a} | 65% |
| **LNP023** | 6.7 | 1.2 | 379 | 2192^{b} | 38% |

| | | | | | |
|---|---|---|---|---|---|
| Note: C!: apparent clearance; T_{1/2}: time required to clear half of the compound; Cₘₐₓ: peak concentration (dose-normalized data in the table); AUC₀₋ₗₐₛₜ: integrated area of concentration during a period from 0 to last sampling time (dose-normalized data in the table); a: a dose of 5 mpk; b: a dose of 30 mpk. | | | | | |

Experimental conclusion: The results of the pharmacokinetic study in rats show that the compounds of the present disclosure have a longer half-life than that of the reference compound, a significantly higher oral plasma exposure than that of the reference compound **LNP023,** and the compounds of the present disclosure have good pharmacokinetic properties.

### Experimental example 4: In vivo PD model of LPS-induced complement activation in mice

Experimental objective: To investigate the inhibitory effect of the compounds of the present disclosure on complement activation in mice induced by LPS stimulation.

Experimental animals: female C57BL/6J mice, 7 to 9 weeks old, weighing 17 to 23 g; supplier: Shanghai Sipple-Bikai Laboratory Animal Co., Ltd.

Experimental procedure: 100 µg of lipopolysaccharide (LPS) in Salmonella typhimurium (Sigma) dissolved in 100 µL of sterile PBS was intraperitoneally injected to induce complement activation in mice. Negative control animals were intraperitoneally injected 100 µL of sterile PBS, and administered alone by tube feeding (PO). Positive control animals were intraperitoneally injected LPS and PO drug vehicles (0.5% (w/v) methyl cellulose and 0.5% (v/v) Tween 80). The time points for administration and plasma collection are as follows:

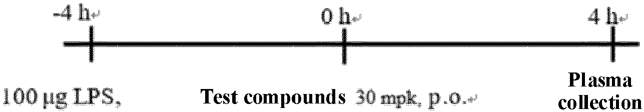

Sample collection: 0.3 mL of blood samples were collected from the orbital venous plexus. All blood samples were added to 1.5 mL commercial EDTA-K2 anticoagulant tubes (supplied by Jiangsu Kangjian Medical Apparatus Co., Ltd.). After collection, within half an hour, the blood sample was centrifuged at 4°C, 3000 g for 10 min to aspirate the supernatant plasma, which was put in dry ice immediately, and stored in a refrigerator at -80°C for Western blot analysis of downstream C3d protein levels after complement activation.

Sample analysis: Mouse plasma (5 µL) + Lysis buffer (27.5 µL) + Loading buffer (12.5 µL) + Reducing buffer (5 µL) were mixed well and incubated at 100°C for 15 min with a loading volume of 5 µL/well, i.e., a loading volume of plasma of 0.5 µL per well.

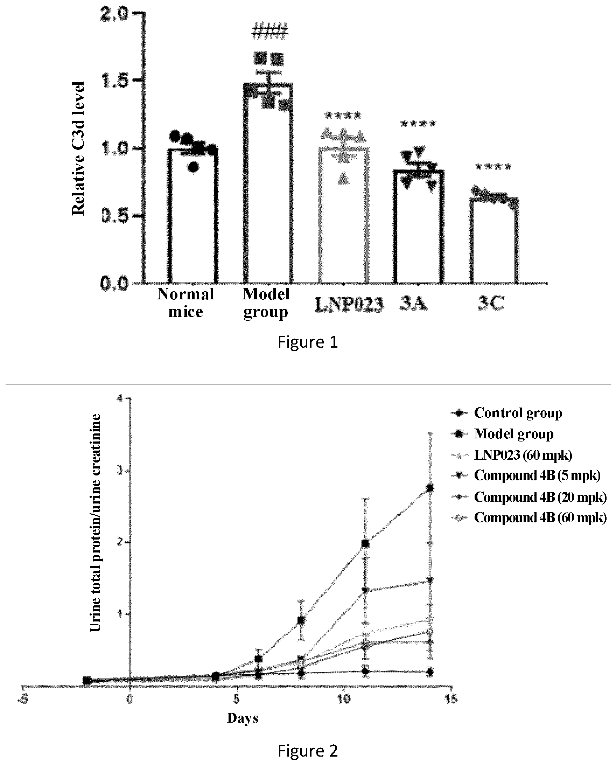

Experimental results: In this experiment, factor B inhibitor **LNP023** from Novartis was used as the reference drug, and the test compounds were the reference compound **LNP023,** compound **3A**, and compound **3C.** All three drugs can significantly inhibit C3d levels, i.e., they can inhibit LPS-induced complement activation, and compound **3A** and compound **3C** have a better inhibitory effect than that of **LNP023.** The specific experimental results are shown in Figure 1, and the data are expressed as: Mean ± SEM, n = 5; ###, p < 0.001, normal group vs model group, t-test; ****, p < 0.0001, administration group vs model group, one-way ANOVA.

Experimental conclusion: The compounds of the present disclosure can significantly inhibit complement activation stimulated by LPS, and have a better inhibitory effect than that of **LNP023.**

### Experimental example 5: In vivo pharmacodynamic model of passive Heymann nephritis in rats

Experimental objective: To investigate the ability of the compounds of the present disclosure to improve renal function of Sheep Anti-Rat Fx1A Serum induced Heymann nephritis in rats, including the evaluation of reducing proteinuria level and improving renal tissue damage.

Experimental animals: male SD rats, 7 to 10 weeks old, weighing 200 to 300 g; supplier: Beijing Vital River Laboratory Animal Technology Co., Ltd.

### Experimental procedure:

### 1. Modeling:

Urine was collected from rats on D-2 before administration. On D1, the first day of the experiment, animals in the control group (group 1) were administered 5 mL/kg of Sheep Non-Immune serum via a single tail vein injection; animals in the model and administration groups (groups 2 to 6) were administered 5 mL/kg of Sheep Anti-Rat Fx1A Serum via a single tail vein injection.

### 2. Administration:

The rats were treated by intragastric administration twice a day with an interval of 8 hours and a volume of 10 mL/kg. On D1, 1 hour before modeling, animals in groups 1 and 2 were administered blank vehicle 20% PEG 400/10% Solutol/70% water; animals in group 3 were administered **LNP023** (60 mpk); animals in groups 4 to 6 were administered different concentrations of compound **4B** (5 mpk, 20 mpk, and 60 mpk); 8 hours after the first administration, each group was administered again with the same dose and volume as the first administration. From D2 to D14, animals in each group were administered different compounds or vehicles for 14 consecutive days (including the first day) according to the dose, volume, administration method, and frequency on D1.

### 3. Sample collection:

Urine collection: Urine samples were collected from rats on D-2 before administration, and 2 to 4 hours and 4 to 6 hours after the first administration on D4, D6, D8, D11, and D14 after administration, transferred to EP tubes and stored in a refrigerator at -80°C to -60°C for the detection of urine protein and creatinine in rats.

Kidney sample collection: All animals were euthanized with CO₂ inhalation anesthesia on Day 15, and bilateral kidneys were collected. The left kidney was cut transversely, and the right kidney was cut longitudinally. Half of the transversely cut kidney (left side) and half of the longitudinally cut kidney (right side) were fixed in formalin (placed in the same EP tube).

### 4. Sample analysis:

(1) Urine creatinine (uCRE) in rats was normally loaded with a 10-fold dilution (10 µL of sample + 90 µL of saline (0.9% saline)) (Decrease mode); if exceeding the upper detection limit, it was 20-fold diluted (5 µL of sample + 95 µL of saline (0.9% saline)) (Increase mode). Urine total protein (uTP) in rats was detected without dilution; if exceeding the upper detection limit, it was 10-fold diluted (10 µL of sample + 90 µL of saline (0.9% saline)) first (Decrease mode); if still exceeding the upper detection limit after 10-fold dilution, it was 100-fold diluted (2 µL of sample + 198 µL of saline (0.9% saline)) (Increase mode). Data were read by the analytical instrument HITACHI LST008 AS(P).
(2) Kidney pathological scoring: The degree of drug damage to rat kidneys (paraffin sections) was analyzed by HE staining. Scoring criteria: 0 means normal, 1 means little cell infiltration in mesangium, 2 means more cell infiltration in mesangium, 3 means proliferation of several mesangial cells and infiltration of several mesangial cells in glomeruli, 4 means urinary casts and atrophy in renal tubule, and formation and sclerosis of glomerular crescent.

Experimental results: Factor B inhibitor **LNP023** from Novartis was used as the reference drug in the detection of urine protein and creatinine in rats. Both **LNP023** and compound **4B** can significantly inhibit urinary protein levels in rats and improve renal function in rats, and compound **4B** is more effective than **LNP023** at a dose of 60 mpk. The specific experimental results are shown in Figure 2, and the data are expressed as: Mean ± SEM, n = 5; ###, p < 0.001, normal group vs model group, t-test; ****, p < 0.0001, administration group vs model group, one-way ANOVA.

The pathological scoring results are shown in Table 4:

**Table 4: Pathological scoring results**

| Group | Pathological score (Mean ± SEM) | | P |
|---|---|---|---|
| | Mean | SEM | |
| Blank group | 0.20 | 0.20 | < 0.05 |
| model group | 1.33 | 0.33 | / |
| Compound **4B**, 5 mg/kg | 0.83 | 0.17 | < 0.05 |
| Compound **4B**, 20 mg/kg | 0.17 | 0.17 | < 0.01 |
| Compound **4B**, 60 mg/kg | 0.33 | 0.21 | < 0.05 |

Experimental conclusion: According to the pathological scoring results, compared with the model group, the compounds of the present disclosure in groups of different doses can significantly improve the degree of renal lesions in the model animals. The compounds of the present disclosure can reduce urine protein levels and improve renal function in rats, and is more effective than **LNP023.**

## Claims

1. A compound represented by formula (I) or a pharmaceutically acceptable salt thereof, 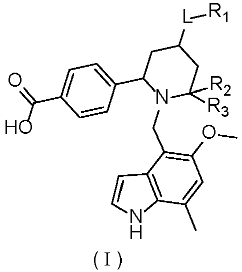 wherein
L is selected from a single bond, NR₄, and O;
R₁ is selected from fluorine, chlorine, C₁₋₅ alkyl, C₃₋₆ cycloalkyl, 3- to 6-membered heterocycloalkyl, -C₁₋₃ alkyl-C₁₋₃ alkoxy, -C₁₋₃ alkyl-C₃₋₆ cycloalkyl, and -C₁₋₃ alkyl-3- to 6-membered heterocycloalkyl, and the C₁₋₅ alkyl, C₃₋₆ cycloalkyl, 3- to 6-membered heterocycloalkyl, -C₁₋₃ alkyl-C₁₋₃ alkoxy, -C₁₋₃ alkyl-C₃₋₆ cycloalkyl, and -C₁₋₃ alkyl-3- to 6-membered heterocycloalkyl are each independently and optionally substituted by 1, 2, or 3 Rₐ groups;
R₂, R₃, and R₄ are each independently selected from H and C₁₋₅ alkyl, and the C₁₋₅ alkyl is optionally substituted by 1, 2, or 3 R_{b} groups;
each Rₐ and R_{b} are independently selected from H, F, Cl, Br, and I;
provided that R₂ and R₃ are not simultaneously selected from H when R₁ is selected from unsubstituted C₁₋₅ alkyl.

2. The compound or the pharmaceutically acceptable salt thereof according to claim 1, which is selected from: 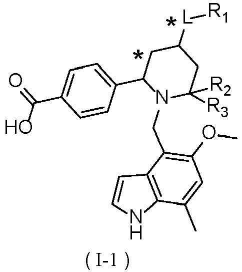 wherein
L, R₁, R₂, and R₃ are as defined in claim 1;
the carbon atom with "*" is a chiral carbon atom, which exists in a (R) or (S) single enantiomer form or a (R) or (S) single enantiomer-rich form.

3. The compound or the pharmaceutically acceptable salt thereof according to claim 2, which is selected from: 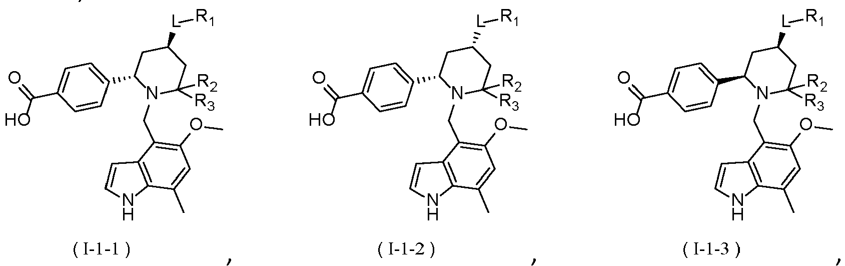 and 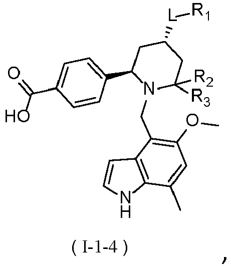 wherein L, R₁, R₂, and R₃ are as defined in claim 2.

4. The compound or the pharmaceutically acceptable salt thereof according to claim 1, wherein R₁ is selected from C₃₋₆ cycloalkyl, 3- to 6-membered heterocycloalkyl, -C₁₋₃ alkyl-C₁₋₃ alkoxy, -C₁₋₃ alkyl-C₃₋₆ cycloalkyl, and -C₁₋₃ alkyl-3- to 6-membered heterocycloalkyl, and the C₃₋₆ cycloalkyl, 3- to 6-membered heterocycloalkyl, -C₁₋₃ alkyl-C₁₋₃ alkoxy, -C₁₋₃ alkyl-C₃₋₆ cycloalkyl, and -C₁₋₃ alkyl-3- to 6-membered heterocycloalkyl are each independently and optionally substituted by 1, 2, or 3 Rₐ groups.

5. The compound or the pharmaceutically acceptable salt thereof according to any one of claims 1 to 3, wherein R₁ is selected from fluorine, chlorine, CH₃, CH₂CH₃, CH₂CH₂CH₃, CH(CH₃)₂, 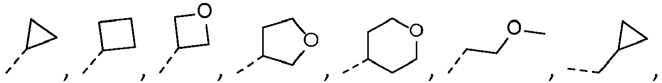 and 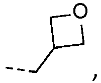 and the CH₃, CH₂CH₃, CH₂CH₂CH₃, CH(CH₃)₂, 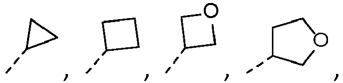 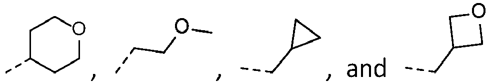 are each independently and optionally substituted by 1, 2, or 3 Rₐ groups.

6. The compound or the pharmaceutically acceptable salt thereof according to claim 5, wherein R₁ is selected from CH₃, CF₃, CH₂CH₃, CH₂CHF₂, CH₂CF₃,

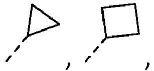

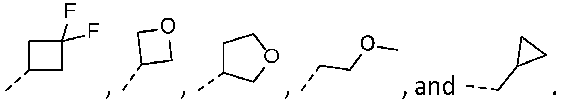

7. The compound or the pharmaceutically acceptable salt thereof according to claim 6, wherein R₁ is selected from 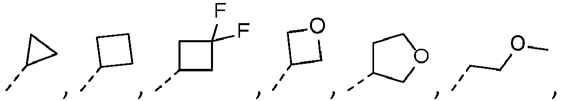 and

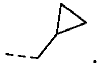

8. The compound or the pharmaceutically acceptable salt thereof according to any one of claims 1 to 3, wherein R₂ is selected from H and CH₃, and the CH₃ is optionally substituted by 1, 2, or 3 R_{b} groups.

9. The compound or the pharmaceutically acceptable salt thereof according to claim 7, wherein R₂ is selected from H, CH₃, CF₃, and CHF₂.

10. The compound or the pharmaceutically acceptable salt thereof according to any one of claims 1 to 3, wherein R₃ is selected from H and CH₃.

11. The compound or the pharmaceutically acceptable salt thereof according to any one of claims 1 to 3, wherein R₄ is selected from H and CH₃.

12. The compound or the pharmaceutically acceptable salt thereof according to any one of claims 1 to 3, wherein L is selected from the single bond and O.

13. The compound or the pharmaceutically acceptable salt thereof according to any one of claims 1 to 3, wherein the structural moiety 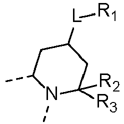 is selected from

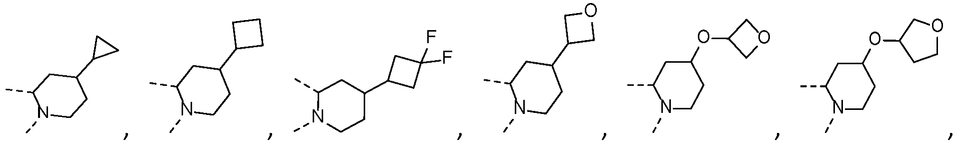

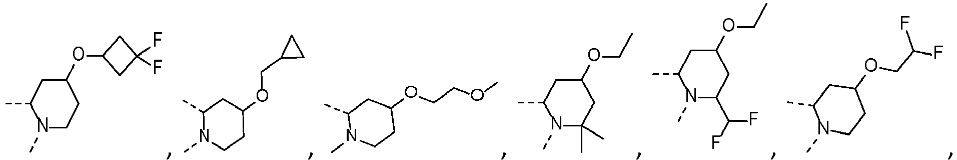

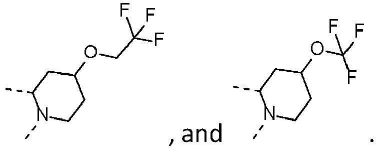

14. The compound or the pharmaceutically acceptable salt thereof according to claim 13, wherein the structural moiety 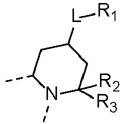 is selected from 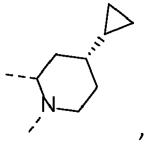 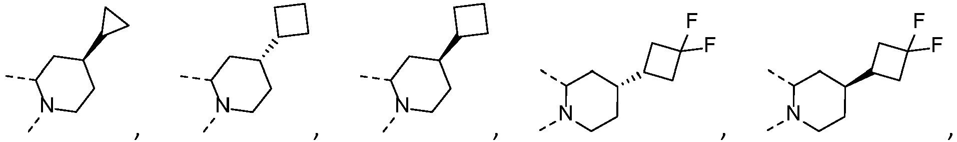 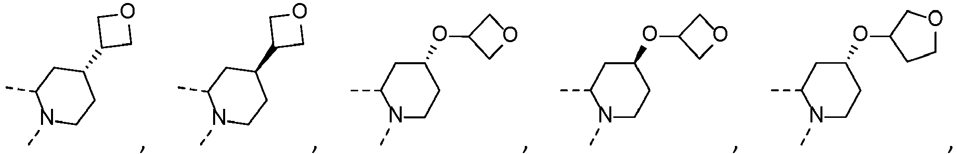 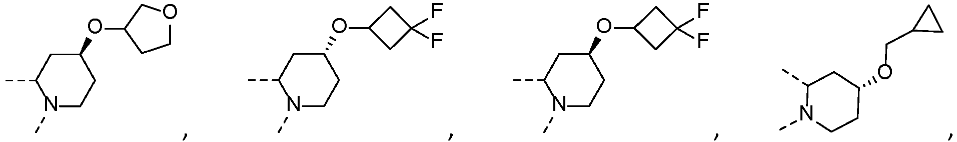 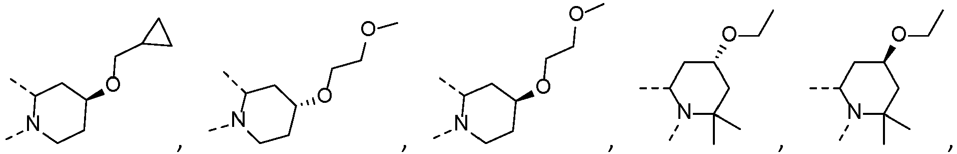 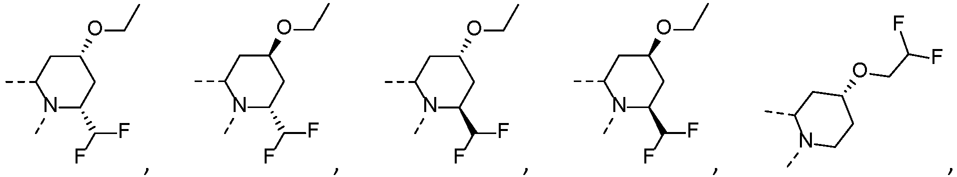 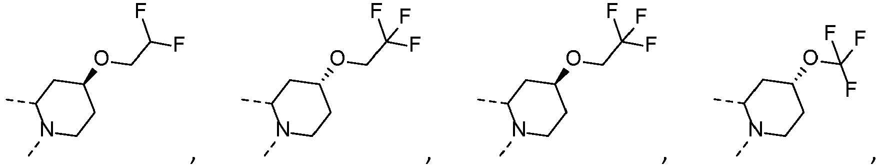 and

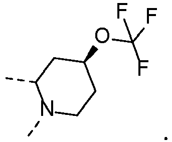

15. A compound represented by the following formulas or a pharmaceutically acceptable salt thereof,

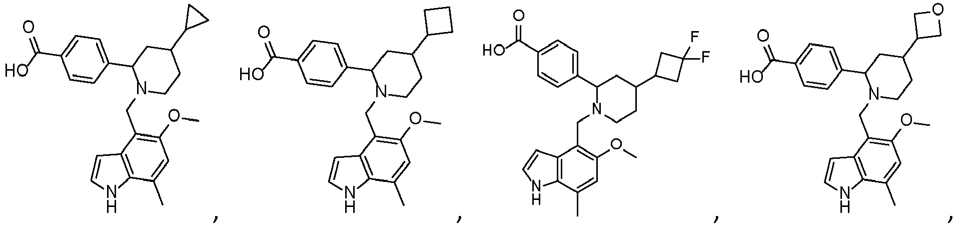

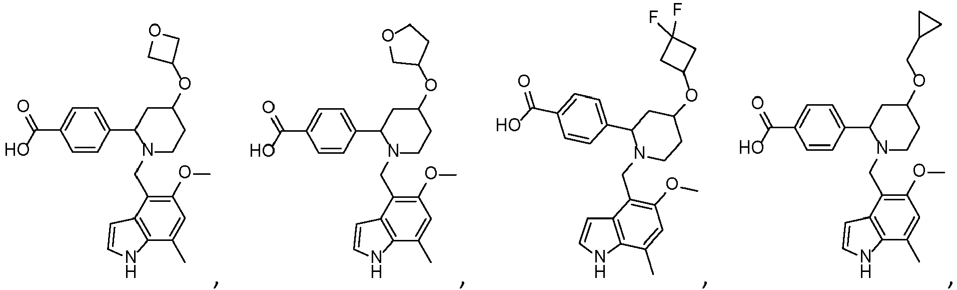

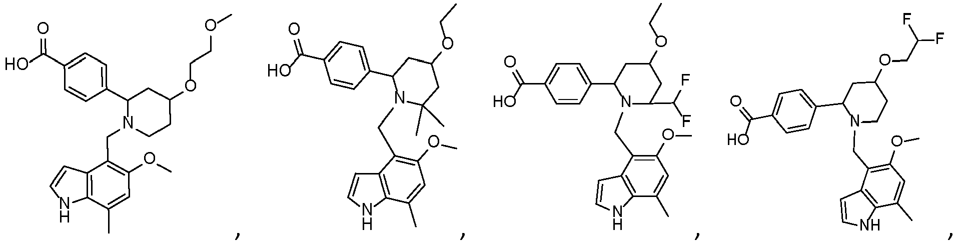

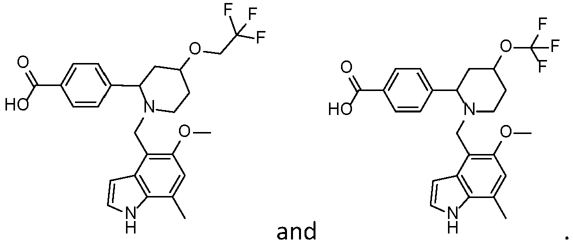

16. The compound or the pharmaceutically acceptable salt thereof according to claim 15, which is selected from:

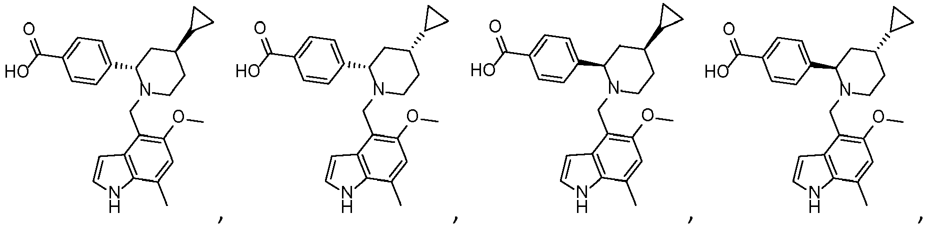

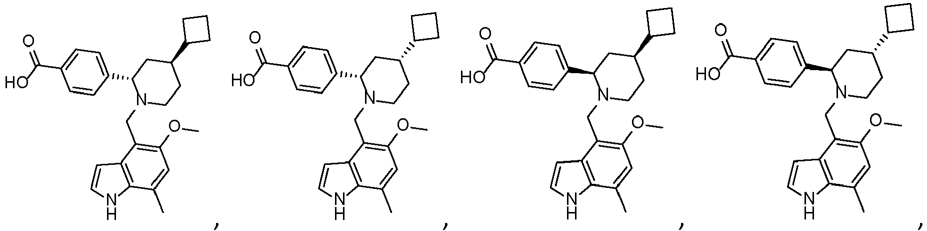

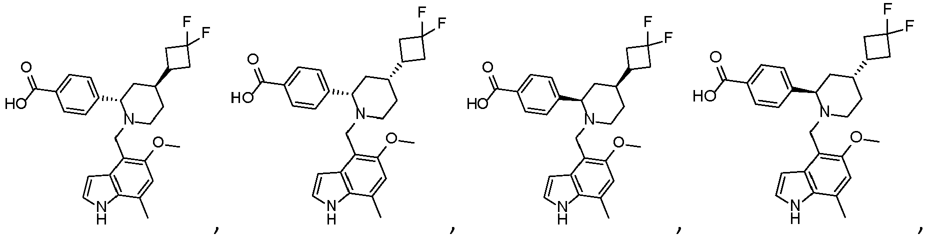

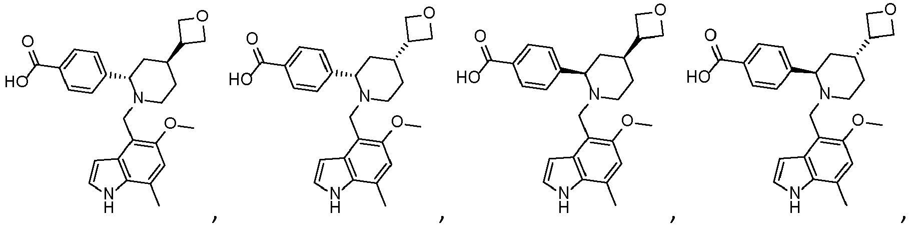

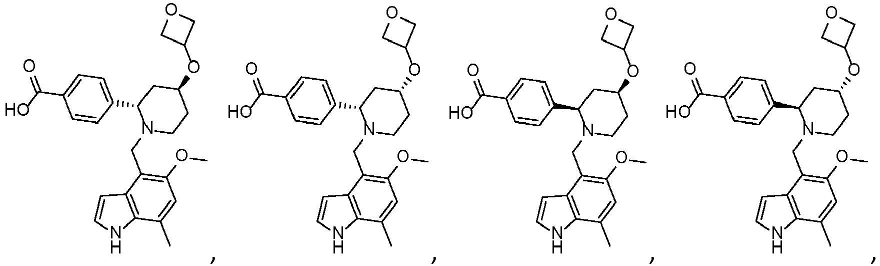

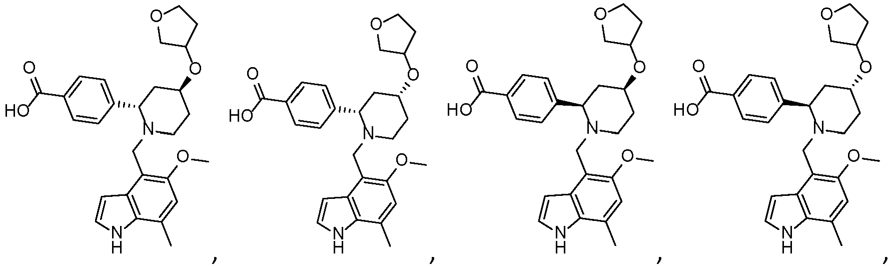

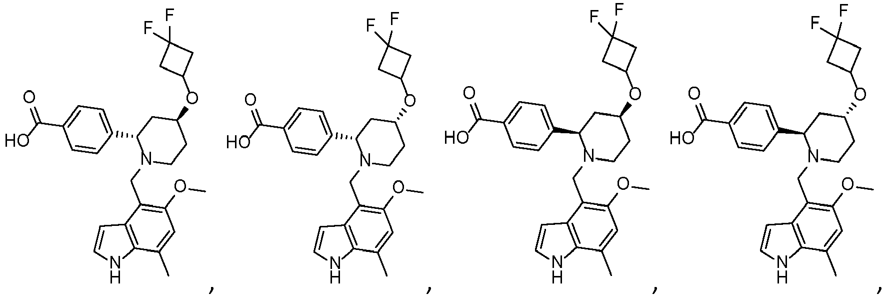

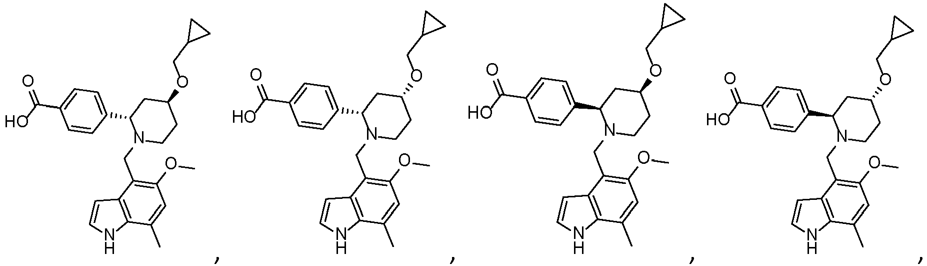

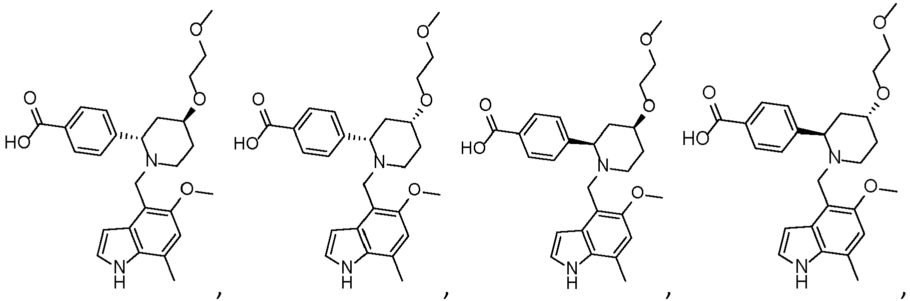

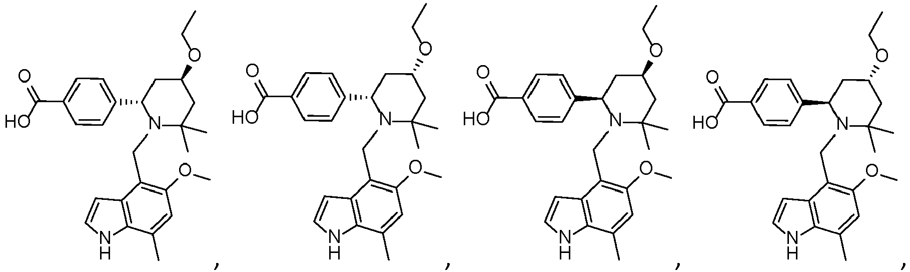

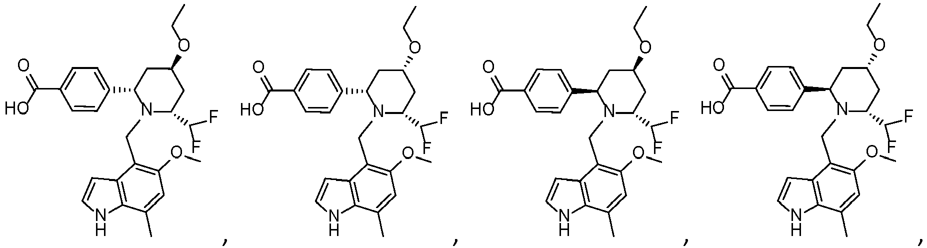

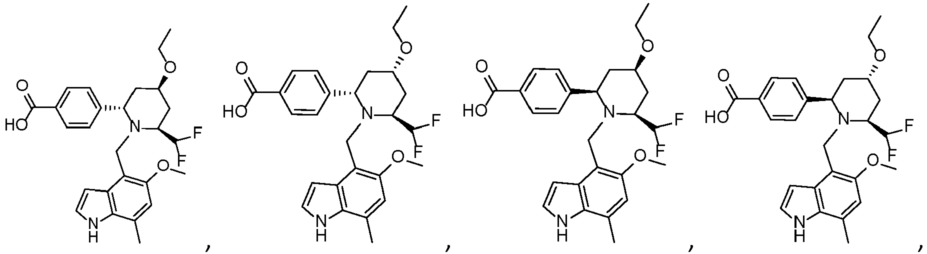

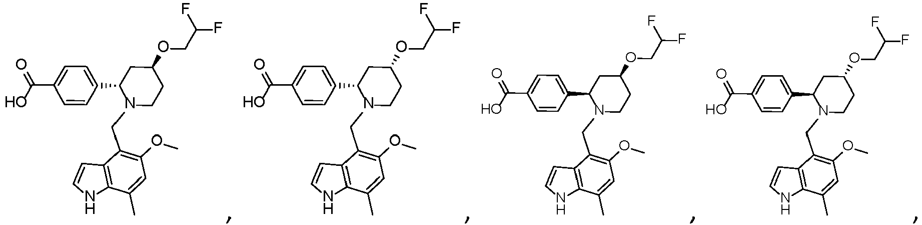

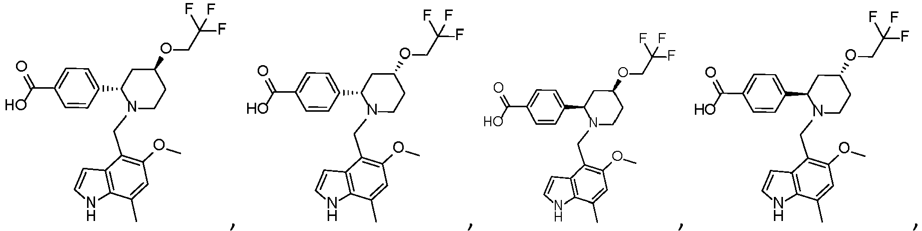

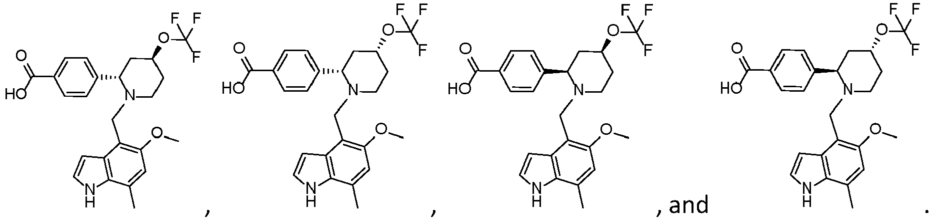

17. A use of the compound or the pharmaceutically acceptable salt thereof according to any one of claims 1 to 16 in the manufacture of a medicament related to complement factor B.
